(19) **Europäisches Patentamt / European Patent Office / Office européen des brevets**

(11) **EP 3 455 669 B1**

(12) # FASCICULE DE BREVET EUROPEEN

(45) Date de publication et mention de la délivrance du brevet:
**20.08.2025 Bulletin 2025/34**

(21) Numéro de dépôt: **17727663.1**

(22) Date de dépôt: **12.05.2017**

(51) Classification Internationale des Brevets (IPC):
**G02C 7/10** (2006.01)     **G02C 7/02** (2006.01)

(52) Classification Coopérative des Brevets (CPC):
**G02C 7/101; G02C 7/027; G02C 7/10; G02C 7/102**

(86) Numéro de dépôt international:
**PCT/FR2017/051159**

(87) Numéro de publication internationale:
**WO 2017/194898 (16.11.2017 Gazette 2017/46)**

(54) **PROCÉDÉ DE FABRICATION D'UN FILTRE POUR UNE LENTILLE OPHTALMIQUE EN FONCTION D'UNE GRANDEUR REPRÉSENTATIVE D'UNE SENSIBILITÉ DYNAMIQUE DE L'OEIL D'UN PORTEUR A UNE VARIATION D'UN FLUX LUMINEUX**

VERFAHREN ZUR HERSTELLUNG EINES FILTERS FÜR EIN BRILLENGLAS IN ABHÄNGIGKEIT VON EINER MENGE, DIE EINE DYNAMISCHE EMPFINDLICHKEIT DES AUGES EINES TRÄGERS GEGENÜBER EINER ÄNDERUNG DES LICHTSTROMS DARSTELLT

METHOD FOR MANUFACTURING A FILTER FOR AN OPHTHALMIC LENS AS A FUNCTION OF A QUANTITY REPRESENTING A DYNAMIC SENSITIVITY OF THE EYE OF A WEARER TO A VARIATION IN A LUMINOUS FLUX

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2016 FR 1654323**

(43) Date de publication de la demande:
**20.03.2019 Bulletin 2019/12**

(73) Titulaire: **Essilor International**
**94220 Charenton-le-Pont (FR)**

(72) Inventeurs:
- **SCHERLEN, Anne-Catherine**
  **94220 Charenton-Le-Pont (FR)**
- **DUBAIL, Marie**
  **94220 Charenton-Le-Pont (FR)**

- **LONGO, Adèle**
  **94220 Charenton-Le-Pont (FR)**
- **BARRAU, Coralie**
  **94220 Charenton-Le-Pont (FR)**
- **MARIE, Sarah**
  **94220 Charenton-Le-Pont (FR)**
- **POLETTO, Elise**
  **94220 Charenton-Le-Pont (FR)**

(74) Mandataire: **Jacobacci Coralis Harle**
**32, rue de l'Arcade**
**75008 Paris (FR)**

(56) Documents cités:
**WO-A1-2013/021102     WO-A1-2014/079574**
**WO-A1-2014/174067     CA-A1- 2 949 250**

**Description**

DOMAINE TECHNIQUE AUQUEL SE RAPPORTE L'INVENTION

La présente invention concerne de manière générale le domaine de l'optique ophtalmique.
Elle concerne plus particulièrement un procédé de fabrication d'un filtre pour une lentille ophthalmique ou d'une lentille ophthalmique comportant un tel filtre, ladite lentille ophtalmique destinée à être placée devant l'œil d'un porteur, ledit filtre étant apte à améliorer ou à maintenir le confort visuel et/ou les performances visuelles dudit porteur.

ARRIERE-PLAN TECHNOLOGIQUE

Il existe des solutions permettant de prescrire à un porteur de lunettes une ou des lentilles ophtalmiques équipées de filtres.
Par exemple, dans le domaine des filtres thérapeutiques, on peut proposer à un porteur différents filtres ou types de filtre en fonction de sa pathologie (cataracte, dégénérescence maculaire, rétinopathie diabétique ...).
La détermination du ou des filtres se fait généralement de manière très empirique, par des tests subjectifs en essayant sur le porteur différentes lentilles ophtalmiques équipées de filtres et en ne retenant que le ou les filtres apportant le plus d'amélioration (voir par exemple Rosenblum et al., « Spectral filters in low-vision correction », Ophthalmic Physiol. Opt. 20 (4), pp. 335-341, 2000).
De tels filtres permettant d'améliorer la vision du contraste et/ou de réduire l'éblouissement en fonction des pathologies sont par exemple proposés par le laboratoire ophtalmique Verbal dans la gamme de lentilles CPF (http://www. verbal.fr/fr/optique-basse-vision).
Le document WO2014/079574 décrit par exemple une paire de lunettes dont l'opacité des verres peut être ajustée en temps réel en fonction d'une mesure du diamètre pupillaire.
Il existe également des solutions permettant de corriger une déficience de la vision des couleurs par le porteur. Le document WO 2001/057583 décrit par exemple une méthode selon laquelle on détermine la réponse spectrale du porteur et on réalise un filtre qui rétablit une vision des couleurs proche de la vision d'un œil normal.
Les procédés de détermination des filtres sont basés sur des méthodes qui sont donc :

- soit subjectives et ne permettent pas d'optimiser le choix des caractéristiques du filtre,
- soit objectives mais limitées à l'amélioration d'une vision des couleurs.

Lors de la détermination d'un filtre, le porteur est souvent confronté à des compromis entre plusieurs critères qu'il doit considérer : variétés d'environnement lumineux, exigence visuelle associée, esthétisme etc....
Les procédés de détermination connus ne permettent pas alors de prendre en compte objectivement la sensibilité du sujet aux caractéristiques d'un environnement lumineux éventuellement dynamique afin de déterminer le filtre destiné à être placé devant l'œil du porteur.
En outre, ils ne permettent pas de prendre en compte la sensibilité dynamique du sujet au flux lumineux, c'est-à-dire l'adaptabilité plus ou moins grande des yeux du porteur à une variation de flux lumineux.

OBJET DE L'INVENTION

Afin de remédier à l'inconvénient précité de l'état de la technique, la présente invention propose un procédé de fabrication d'un filtre pour une lentille ophtalmique ou d'une lentille ophtalmique comportant un tel filtre selon la revendication 1.
Ainsi, grâce au procédé selon l'invention, la sensibilité dynamique de l'œil ou des yeux du porteur aux variations de flux lumineux est déterminée objectivement ou subjectivement, afin de paramétrer au moins une caractéristique optique du filtre pour optimiser les performances visuelles et/ou le confort visuel du porteur dans une tâche donnée. Le filtre est ainsi personnalisé pour le porteur.
On entend ici par sensibilité dynamique la capacité d'adaptation de l'œil ou des yeux à une évolution du flux lumineux perçu, présentant par exemple une augmentation ou une diminution d'éclairement d'au moins 10 lux sur un intervalle de temps compris par exemple entre 0,1 et 60 secondes, pour un éclairement initial compris entre 0 et 1000 lux.
La grandeur représentative de la sensibilité dynamique de l'œil du porteur à la variation du flux lumineux est représentative de l'évolution du confort visuel et/ou des performances visuelles du porteur en fonction de la variation du flux lumineux.
Cette variation du flux lumineux peut correspondre à une modification de l'intensité ou du spectre de longueurs d'onde du flux lumineux dans le temps, ou encore à une modification spatiale du flux lumineux, par exemple un déplacement rapide de la source lumineuse.

Ces performances visuelles et le confort visuel peuvent être limités à la fois par une sensibilité dynamique insuffisante du porteur au flux lumineux, et à la fois par les caractéristiques mêmes du filtre.

Selon la précision visuelle exigée par le porteur et ses capacités d'adaptation aux variations de flux lumineux, on adaptera spécifiquement les paramètres du filtre.

Selon un des aspects de l'invention, la variation de flux lumineux peut correspondre :

- soit à une variation de flux lumineux « réel » auquel est soumis le porteur dans la tâche donnée ; en d'autres termes le flux lumineux caractéristique est caractéristique de l'environnement lumineux ambiant dans lequel le porteur se retrouvera pour la réalisation de la tâche visuelle ;
- soit à une variation de flux lumineux « artificiel » en ce sens qu'il reproduit au moins partiellement le flux lumineux auquel sera soumis le porteur, et est représentatif d'au moins une source lumineuse d'inconfort visuel ou de perte de performances visuelles pour le porteur.

Bien entendu, on peut envisager de déterminer, à l'étape a), une pluralité de grandeurs représentatives de la sensibilité dynamique de l'œil ou des deux yeux du porteur à une variation d'un flux lumineux, et de prendre en compte, à l'étape b), une combinaison de ces grandeurs représentatives pour déterminer ladite caractéristique optique dudit filtre.

Selon un premier aspect de l'invention ladite étape a) de détermination de la grandeur représentative de la sensibilité dynamique de l'œil du porteur à la variation de flux lumineux comprend :

a1) une étape de soumission du porteur à ladite variation de flux lumineux, et
a2) une étape de mesure d'une grandeur relative à l'adaptation de l'œil à cette variation de flux lumineux, réalisée sur le porteur soumis à ladite variation de flux lumineux.

L'étape de soumission du porteur à la variation de flux lumineux correspond soit à la mise en situation du porteur dans l'environnement lumineux dans lequel il sera susceptible d'effectuer une certaine tâche visuelle, soit à la reproduction, au moins partielle, de cet environnement lumineux par un flux lumineux caractéristique contrôlé de manière à se rapprocher au plus près de la situation réelle du porteur.

Anatomiquement et physiologiquement, plusieurs composantes de l'œil du porteur interagissent dans la gestion de la variation de flux lumineux. Afin de déterminer le filtre adéquat, il est utile de tenir compte de l'ensemble des caractéristiques physiologiques de l'œil du porteur et/ou des structures connexes des yeux prenant en charge cette variation de flux lumineux (analyse multiparamétrique). En fonction de la capacité ou de la fragilité de cet œil, le filtre déterminé devra soulager ledit œil de la composante de la variation lumineuse non gérée de manière optimale ou adéquate pour un état donné de l'œil.

On comprendra par ailleurs qu'il sera utile de caractériser ladite variation de flux lumineux à l'aide d'un ensemble de capteurs, tels que spectromètres, luxmètres, etc..., permettant de mesurer les propriétés optiques et photométriques des sources lumineuses dans l'environnement du porteur.

Il est également possible de déterminer par simulation ou calcul optiques les caractéristiques de la variation de flux lumineux.

On répète de préférence l'étape a1) pour différentes intensités initiales du flux lumineux.

A l'étape a2), on réalise des mesures relatives à l'un ou aux deux yeux du porteur soumis à la variation du flux lumineux.

Dans certains modes de réalisation, la grandeur représentative de la sensibilité dynamique de l'œil du porteur à ladite variation de flux lumineux est choisie parmi l'une au moins des grandeurs suivantes :

- une grandeur de mesure objective physiologique du porteur,
- une grandeur de mesure objective physique du porteur,
- une grandeur de mesure subjective liée à la perception ou à l'expression du porteur.

Par « grandeur de mesure objective physiologique » du porteur, on entend toutes valeurs relatives à la mesure d'au moins un paramètre ou d'au moins une caractéristique lié à l'intégrité et au fonctionnement d'une composante du système oculaire ou des structures connexes à ce système. Le choix d'une telle grandeur représentative permet d'évaluer les capacités physiologiques de l'œil ou des éléments connexes à traiter un ensemble ou une partie des caractéristiques du flux lumineux. Cette analyse permet d'identifier les conditions ou situations à partir desquelles le porteur ne pourra pas gérer naturellement le flux lumineux. La prescription d'un filtre permettra alors de compenser la perte de vision et/ou de confort visuel associée.

Par « grandeur de mesure objective physique » du porteur, on entend toute valeur relative à la mesure d'un moins un paramètre caractéristique d'un état de la structure et des fonctions oculaires ou des structures connexes par une mesure optique et/ou photométrique. L'ajout d'une instrumentation physique permet de caractériser et de quantifier de manière inférentielle une composante de la structure oculaire ou connexe. Le choix d'une telle grandeur représentative permet de quantifier par une mesure physique les capacités et les performances d'une ou plusieurs structures oculaires ou connexes en lien avec les processus d'éblouissement. Selon la structure étudiée et les résultats obtenus, on orientera différemment les caractéristiques du filtre pour optimiser le confort et ou les performances visuelles selon la ou les fragilités de la structure oculaire et connexe considérée.

Par « grandeur de mesure subjective liée à la perception ou à l'expression » du porteur, on entend toutes les réponses exprimées par le porteur à travers soit un questionnaire ou à des questions en lien avec des tests réalisés dans lequel le porteur doit exprimer ce qu'il a perçu ou ressenti visuellement. Le choix d'une telle grandeur représentative permet de déterminer subjectivement des performances visuelles et ou des inconforts visuels ressentis et exprimés par le porteur. Cette évaluation permet de définir les conditions ou situations dans lesquels le porteur obtient une performance visuelle optimale/ou un confort optimal et également les conditions d'inconfort et de perte de performance visuelle.

Plus particulièrement, selon certains aspects de la réalisation du procédé selon l'invention :

- à l'étape a1), on soumet le porteur à un flux lumineux prédéterminé pendant une première phase d'exposition, puis on place le porteur dans l'obscurité lors d'une deuxième phase d'obscurité et, à l'étape a2), on mesure une sensibilité moyenne pendant une période de temps déterminée après le début de la deuxième phase et/ou un temps d'adaptation à l'obscurité correspondant au temps nécessaire pour que la sensibilité à la lumière des yeux du porteur retrouve une valeur de sensibilité prédéterminée et/ou
- à l'étape a2), on détermine la variation de taille de la pupille au cours du temps pendant au moins ladite variation de flux lumineux de l'étape a1).

On peut également déterminer la variation de taille de la pupille au cours du temps pendant l'étape a2) de retour à l'obscurité.

Selon un deuxième aspect du procédé selon l'invention, ladite étape a) de détermination de la grandeur représentative de la sensibilité dynamique de l'œil du porteur à la variation du flux lumineux caractéristique comprend :

a3) une étape de soumission du porteur à un questionnaire permettant d'apprécier la sensibilité du porteur à ladite variation de flux lumineux,
a4) une étape de collecte des réponses du porteur audit questionnaire.

Ainsi, ce questionnaire comporte par exemple une ou plusieurs questions posées au porteur sur les différentes caractéristiques des variations de flux lumineux auxquels il est ou sera confronté, et pour lesquels un inconfort visuel ou une perte de performances visuelles est rapporté.

Selon certains aspects du procédé selon l'invention, à l'étape a), la variation du flux lumineux comprend au moins :

- une variation temporelle et/ou spatiale d'une intensité dudit flux lumineux et/ou
- une variation temporelle et/ou spatiale d'un spectre dudit flux lumineux et/ou
- une variation dans l'espace d'une distribution spatiale dudit flux lumineux et/ou
- une variation dans l'espace d'une distribution angulaire dudit flux lumineux ;

Lorsque le flux lumineux est issu d'une ou plusieurs sources lumineuses, la distribution spatiale dudit flux lumineux caractéristique correspond, par exemple, à la donnée de l'étendue spatiale de la ou des sources (source ponctuelle, source étendue). La distribution angulaire quant à elle correspond, par exemple, à la donnée du diagramme angulaire d'émission (source directive/collimatée, source non directive, etc...).

Selon certains aspects du procédé selon l'invention,

- la variation temporelle d'intensité du flux lumineux est réalisée avec un profil de variation temporelle donné, et/ou une vitesse de variation temporelle donnée, et/ou une amplitude de variation donné et/ou une intensité de flux lumineux initiale et/ou finale donnée ;
- à l'étape a), on soumet le porteur à différentes variations temporelles d'intensité du flux lumineux, présentant différents profils de variation temporelle donnés, et/ou différentes vitesses de variation temporelle données, et/ou différentes amplitudes de variation donnés et/ou différentes intensités de flux lumineux initiales et/ou finales données.

Selon d'autres aspects du procédé selon l'invention :

- à l'étape b), au moins une autre caractéristique optique du filtre déterminée consiste en :

- le taux d'absorption et/ ou de transmission et/ou de réflexion et/ou de coupure dudit filtre,
- la réponse spectrale dudit filtre,
- la répartition spatiale de ces caractéristiques sur ladite lentille ophtalmique,
- les propriétés photochromiques ou électrochromiques du filtre.

Le taux de coupure du filtre peut être mesuré en utilisant la méthode décrite par exemple dans la norme ISO 8980-3:2003 *« Transmittance specification and test methods »*.

La réponse spectrale du filtre peut quant à elle correspondre à la réflectance $R(\lambda)$ ou à la transmittance $T(\lambda)$, par exemple mesurées au moyen d'un spectromètre utilisant un illuminant standardisé D65.

Dans un mode de réalisation particulier, la caractéristique optique du filtre est déterminée également en fonction d'un indicateur sur le flux lumineux et/ou du besoin visuel auquel sera soumis le porteur dans ses activités.

Certaines caractéristiques avantageuses de l'invention sont définies dans les revendications dépendantes 2 à 16.

En particulier, ladite grandeur représentative de la sensibilité dynamique de l'œil du porteur à la variation du flux lumineux correspond à un temps d'adaptation de l'œil à la variation de ce flux lumineux. Il peut s'agir notamment d'un temps de récupération des performances de l'œil après une diminution de l'intensité du flux lumineux ou un temps de latence de la pupille après une augmentation de l'intensité du flux lumineux, qui seront décrits plus en détails ultérieurement.

Ladite grandeur représentative de la sensibilité dynamique de l'œil du porteur à la variation du flux lumineux peut également correspondre à une mesure du niveau lumineux à partir duquel une baisse des performances visuelles et / ou du confort visuel est observée.

Quel que soit le type de filtre envisagé précédemment, on détermine de préférence la transmission optique du filtre ou le niveau de transmission de l'un des états clair ou foncé du filtre à au moins une longueur d'onde donnée, de préférence sur au moins un intervalle de longueur d'onde donné.

De manière similaire, on détermine la transmission optique du filtre ou le niveau de transmission de l'un des états clair ou foncé du filtre sur au moins une zone spatiale donnée du filtre.

Ladite zone spatiale du filtre peut par exemple être une zone centrale ou périphérique, supérieure ou inférieure, une zone de vision de prés, de loin ou intermédiaire, une zone centrée sur la direction de regard du porteur.

Selon certains aspects du procédé selon l'invention, ladite grandeur représentative de la sensibilité dynamique de l'œil du porteur à la variation du flux lumineux est déterminée en tenant compte d'au moins un des paramètres suivants :

- un paramètre relatif aux habitudes d'exposition à la lumière du porteur passées, présentes et/ou futures : par exemple un éclairement moyen initial de l'œil avant variation du flux lumineux, activités pratiquées/métier, saison, situation géographique, lumière artificielle ou naturelle, durée d'exposition...,
- un paramètre relatif à la sensibilité statique au flux lumineux du porteur : par « sensibilité à la lumière » du porteur, on entend toute réaction ou modification d'un confort ou d'une performance visuelle plus ou moins intense et prolongée liée à un flux ou stimuli lumineux temporaires ou continus,
- un paramètre relatif à une amplitude de la variation temporelle et/ou spatiale d'intensité et/ou de spectre du flux lumineux,
- un paramètre subjectif relatif aux performances visuelles du porteur pour des conditions lumineuses et/ou de variation lumineuse données,
- un paramètre subjectif relatif au confort visuel pour des conditions lumineuses et/ou de variations lumineuses données,
- un paramètre lié à l'âge du porteur,
- un paramètre relatif à l'utilisation de lunettes de soleil,
- un paramètre lié à un coefficient de diffusion intraoculaire de l'œil du porteur,
- un paramètre lié à une densité et/ou à une répartition du pigment maculaire de l'œil du porteur,
- un paramètre lié à une capacité d'adaptation de la rétine à la lumière ou à l'obscurité,
- un paramètre lié à une dynamique de réponse pupillaire à la variation lumineuse incluant le temps de latence, l'amplitude de constriction, la vitesse de constriction, le temps de récupération de la pupille après arrêt de l'illumination
- un paramètre relatif à une pathologie visuelle ou une anomalie oculaire éventuelle du porteur, par exemple la présence d'un défaut diffractif ou d'un cristallin artificiel suite à une opération de la cataracte
- un paramètre lié à un seuil de variation du confort visuel et/ou des performances visuelles exprimé ou mesuré: par exemple un temps de récupération des performances visuelles après un passage brusque de la lumière à l'obscurité ou inversement ;
- ladite étape a) comprend une étape de mesure du flux lumineux dynamique auquel est habituellement soumis le porteur ;
- ladite étape de mesure du flux lumineux est réalisée à l'aide d'un capteur de flux lumineux indépendant ou intégré à une paire de lunettes ou un objet connecté du porteur.

Selon un autre aspect du procédé de l'invention, on réalise en outre une étape de détermination d'une grandeur représentative de l'environnement dans lequel le filtre est utilisé par le porteur et ladite caractéristique optique dudit filtre est déterminée tenant compte de cette grandeur représentative de l'environnement.

Cette grandeur représentative de l'environnement est par exemple relative au lieu géographique, incluant altitude, longitude, latitude, pays... ou à la saison de l'année, qui est lié à l'ensoleillement et donc à l'intensité moyenne du flux lumineux perçu par le porteur à l'extérieur. Il peut également s'agir d'une grandeur représentative de la fraction de temps passé à l'extérieur.

Grâce à cette détermination, il est possible de déterminer des caractéristiques optiques d'un filtre du type électrochrome ou photochrome en commandant ces caractéristiques de manière à limiter les variations de flux lumineux de telle sorte que la vitesse des variations et/ou l'amplitude de ces variations et/ou les intensités initiales et finales restent en dessous de valeurs seuil de confort.

DESCRIPTION DETAILLEE D'UN EXEMPLE DE REALISATION

On propose plus précisément de détailler ci-après quatre exemples de procédé de détermination d'un filtre conforme à l'invention, pris isolément ou combinés, dans lesquels :

- l'exemple 1 concerne la détermination d'un filtre en fonction de la densité du pigment maculaire ;
- l'exemple 2 concerne la détermination d'un filtre en fonction de l'adaptation de la rétine à l'obscurité et/ou lumière ;
- l'exemple 3 concerne la détermination d'un filtre en fonction de la réponse pupillaire à la variation de flux lumineux ;
- l'exemple 4 concerne la détermination d'un filtre en fonction d'un cône de prescription,
- l'exemple 5 concerne la détermination d'un filtre à partir d'un questionnaire permettant de déterminer la sensibilité dynamique de l'œil du porteur à une variation d'un flux lumineux.

Les procédés décrits ci-après peuvent être considérés individuellement ou bien être couplés.

La description des exemples qui va suivre en regard des dessins annexés, donnés à titre d'exemples non limitatifs, fera bien comprendre en quoi consiste l'invention et comment elle peut être réalisée.

Sur les dessins annexés :

- la figure 1 représente les courbes de variation en fonction du temps de la sensibilité de l'œil d'un porteur après une variation de flux lumineux, pendant les 5 premières minutes après cette variation, avec et sans filtre, dans l'exemple 2;
- la figure 2 représente une courbe de variation en fonction du temps de la sensibilité de l'œil d'un porteur après une variation de flux lumineux similaires aux courbes de la figure 1, pendant les 30 premières minutes après cette variation;
- la figure 3 représente le taux de transmission d'un filtre photochromique dans un état clair (courbe CT1) et dans un état sombre (courbe CT2) ;
- les figures 4 et 5 représentent les résultats expérimentaux de sensibilité moyenne en dB et de temps de récupération moyenne en secondes pour un groupe de porteur muni d'une lentille ophtalmique filtrante (résultats T1) ou non (résultats R) ;
- la figure 6 représente un temps de latence de la pupille en fonction d'un niveau de confort visuel pour différents porteurs, regroupés en fonction de leurs âges (exemple 3);
- la figure 7 donne un exemple de cône de prescription déterminé dans l'exemple 4 ;
- la figure 8 montre l'influence du profil de la variation d'éclairement du flux lumineux sur la valeur seuil d'éclairement de confort du porteur ;
- la figure 9 montre l'influence de l'éclairement initial du flux lumineux avant variation sur la latitude d'adaptation de l'œil du porteur ;
- les figures 10 et 11 montrent la variation d'un indicateur de confort visuel de deux porteurs différents en fonction d'une variation d'éclairement du flux lumineux ;
- la figure 12 représente un autre profil de variation du flux lumineux auquel est soumis le porteur dans l'exemple 4;
- les figures 13 et 14 montrent la variation du temps de récupération des performances visuelles en fonction de la variation d'éclairement du flux lumineux de la figure 12 pour deux porteurs différents.

La variation de flux lumineux peut impacter différemment les performances visuelles et le confort selon les porteurs. Avantageusement, grâce au procédé selon l'invention, le filtre est déterminé pour préserver au mieux les performances visuelles et le confort du porteur en cas de variation de flux lumineux.

L'invention dans sa généralité concerne un procédé de fabrication d'un filtre pour une lentille ophthalmique ou d'une lentille ophtalmique comportant un tel filtre selon la revendication 1.

De préférence, ladite grandeur représentative de la sensibilité dynamique de l'œil du porteur à la variation du flux lumineux est représentative de l'évolution du confort visuel et/ou des performances visuelles du porteur en fonction de la variation du flux lumineux.

On parlera dans la suite de différentes caractéristiques photométriques du flux lumineux, notamment de :

- son intensité lumineuse en candela dans une direction donnée, qui correspond au flux lumineux émis ramené à l'angle solide unité centré sur cette direction,
- la luminance de la source lumineuse émettant le flux, égale au flux lumineux émis ramené à l'angle solide unité et à l'aire apparente (cosinus) unité dans une direction d'observation donnée, en candela par mètre carré,
- l'éclairement, égal au flux lumineux reçu par unité de surface, en lux.

De manière générale, la luminance de la source et l'éclairement du flux lumineux sont liés à son intensité et on parlera de manière générale de l'intensité du flux lumineux. On comprend qu'une variation de l'intensité se traduit en général par une variation de luminance (si la source est la même) et d'éclairement.

La variation du flux lumineux comprend au moins:

- une variation temporelle et/ou spatiale d'une intensité dudit flux lumineux et/ou
- une variation temporelle et/ou spatiale d'un spectre dudit flux lumineux et/ou

- une variation dans l'espace d'une distribution spatiale dudit flux lumineux et/ou
- une variation dans l'espace d'une distribution angulaire dudit flux lumineux.

Dans le cas où la variation de flux lumineux concerne une variation de l'intensité du flux lumineux, il peut évidemment s'agir d'une variation positive ou négative d'intensité, c'est-à-dire d'une augmentation ou d'une diminution de l'intensité du flux lumineux.

Toutes les conditions de variation du flux lumineux ont ici prises en compte, notamment les intensités initiales et finales du flux lumineux et la vitesse de variation du flux lumineux.

Dans le cas d'une variation temporelle du spectre, ce sont les valeurs de transmission en fonction de chaque longueur d'onde qui varie. La transmission moyenne peut en revanche rester la même.

La variation temporelle d'intensité du flux lumineux est réalisée avec un profil de variation temporelle donné, et/ou une vitesse de variation temporelle donnée, et/ou une amplitude de variation donnée et/ou une intensité de flux lumineux initiale et/ou finale donnée.

De manière générale, à l'étape a), on soumet le porteur à différentes variations temporelles d'intensité du flux lumineux, présentant différents profils de variation temporelle donnés, et/ou différentes vitesses de variation temporelle données, et/ou différentes amplitudes de variation donnés et/ou différentes intensités de flux lumineux initiales données.

La caractéristique optique du filtre déterminée à l'étape b) peut notamment être :

- la transmission de ce filtre à au moins une longueur d'onde donnée, sur au moins une zone spatiale du filtre donnée,
- le taux de coupure (l'absorption ou la réflectance) de ce filtre à au moins une longueur d'onde donnée, sur au moins une zone spatiale du filtre donnée,
- le temps de passage de l'un à l'autre d'un état clair et d'un état foncé pour les filtres présentant des propriétés photochromiques ou électrochromiques avec au moins deux états clair et foncé associés respectivement à une première valeur de transmission de la lumière à une longueur d'onde donnée, de préférence sur un domaine de longueurs d'onde prédéterminé et à une deuxième valeur de transmission de la lumière à cette longueur d'onde donnée ou sur ce domaine de longueurs d'onde prédéterminé, inférieure à la première.

La transmission $T(\lambda)$ du filtre à une longueur d'onde $\lambda$ donnée est donnée par le rapport entre l'intensité $I$ du flux lumineux transmis par le filtre et l'intensité $I_0$ du flux lumineux incident sur le filtre : $T(\lambda) = I / I_0$.

Cette transmission est comprise entre 0 ou 1 ou exprimée en pourcentage.

Cette transmission peut résulter aussi bien du passage du flux lumineux à travers un filtre par absorption que par un filtre interférentiel.

Pour un filtre par absorption, l'absorption $A(\lambda)$ du filtre est égale à un moins la transmission du filtre :

$$A(\lambda) = 1 - T(\lambda),$$

soit A(λ) = 100%- T(λ) en pourcentage.

Pour un filtre interférentiel, la réflectance R(λ) du filtre est égale à un moins la transmission du filtre.

Dans la suite, sauf mention contraire, le terme de « transmission » ou « absorption » renvoie indifféremment à une transmission ou absorption moyenne du filtre sur l'ensemble du spectre du flux lumineux incident considéré ou à un spectre de transmission ou absorption présentant différentes valeurs sur un intervalle de longueur d'onde d'intérêt.

Comme cela sera apparent à la lecture des exemples suivants, de manière générale, à l'étape b), on détermine une transmission optique du filtre à au moins une longueur d'onde, sur au moins une zone spatiale de ce filtre, d'autant plus faible que la grandeur représentative de la sensibilité dynamique de l'œil du porteur déterminée à l'étape a) indique une capacité d'adaptation faible vis-à-vis d'une augmentation, aussi appelé variation positive, de l'intensité du flux lumineux.

De manière similaire, à l'étape b), on détermine une transmission optique du filtre à au moins une longueur d'onde, sur au moins une zone spatiale de ce filtre, d'autant plus grande que la grandeur représentative de la sensibilité dynamique de l'œil du porteur déterminée à l'étape a) indique une capacité d'adaptation faible vis-à-vis d'une diminution, aussi appelée variation négative, de l'intensité du flux lumineux.

En outre, de préférence, à l'étape b), on détermine la transmission optique du filtre, à au moins une longueur d'onde, sur au moins une zone spatiale de ce filtre, en prenant en compte la sensibilité dynamique du porteur vis-à-vis des variations positives et négatives de l'intensité du flux lumineux, c'est-à-dire la grandeur représentative de cette sensibilité dynamique déterminée à l'étape a).

La valeur de la transmission déterminée représente alors un compromis optimal en fonction de la sensibilité dynamique du porteur.

En particulier, à l'étape a), ladite grandeur représentative de la sensibilité dynamique de l'œil du porteur à la variation du flux lumineux peut comprendre une vitesse seuil de confort du porteur pour la variation de flux lumineux et/ou une valeur seuil de confort pour l'intensité lumineuse perçue par le porteur lors de la variation de flux lumineux et, à l'étape b), la transmission optique du filtre, à au moins une longueur d'onde, sur au moins une zone spatiale de ce filtre, est déterminée en prenant en compte cette vitesse seuil de confort du porteur pour la variation de flux lumineux et/ou cette valeur seuil de confort pour l''intensité lumineuse perçue par le porteur lors de la variation de flux lumineux.

Lorsque le filtre présente des propriétés photochromiques ou électrochromiques autorisant le passage de l'un à l'autre d'un état clair à un état foncé du filtre correspondant à au moins deux niveaux différents de transmission de la lumière à au moins une longueur d'onde, et, à l'étape b), on détermine le niveau de transmission d'au moins un desdits états clair et foncé en fonction de la sensibilité dynamique du porteur aux variations de flux lumineux et/ou une durée nécessaire pour passer de l'un à l'autre des états clair et foncé d'autant plus courte que la grandeur représentative de la sensibilité dynamique de l'œil du porteur déterminée à l'étape a) indique une capacité d'adaptation faible vis-à-vis des variations négatives de l'intensité du flux lumineux.

De manière générale, si le porteur est plus gêné par des variations d'intensité lumineuses croissante, on lui propose un filtre photochromique avec un passage à l'état foncé rapide. Si le porteur est plus gêné par des variations d'intensité lumineuses décroissante, on lui propose un filtre photochromique avec passage à l'état clair rapide.

Par exemple, ladite grandeur représentative de la sensibilité dynamique de l'œil du porteur à la variation du flux lumineux correspond à un temps d'adaptation de l'œil à la variation de ce flux lumineux (voir exemples 2 et 3).

Ce temps d'adaptation est par exemple un temps de récupération des performances de l'œil ou un temps de latence de la pupille.

Le temps de récupération est le temps nécessaire pour retrouver un confort et/ou une performance initiale de l'œil. Il correspond au temps nécessaire à la régénération des pigments des photorécepteurs après une variation de flux lumineux ou un niveau d'éclairement saturant la rétine, lors d'un retour à l'obscurité.

Le temps de latence (ou temps de réaction) de la pupille est le temps nécessaire pour que la pupille adapte sa taille suite à un changement de flux lumineux, aussi bien dans le cas d'une augmentation ou d'une diminution de l'intensité du flux lumineux.

Selon un autre exemple (voir exemple 4), le filtre présentant des propriétés photochromiques ou électrochromiques, à l'étape a), ladite grandeur représentative de la sensibilité dynamique de l'œil du porteur à la variation du flux lumineux correspond à une vitesse seuil de confort du porteur et/ou une variation de seuil de confort pour la variation de flux lumineux et, à l'étape b), un écart de transmission entre les états clair et foncé, et/ou une durée de passage entre ces deux état et/ou une vitesse de passage de l'un à l'autre des états clair et foncé du filtre est déterminée en fonction de cette vitesse seuil de confort et/ou une variation de seuil de confort.

Alternativement, à l'étape a), ladite grandeur représentative de la sensibilité dynamique de l'œil du porteur à la variation du flux lumineux correspond à une valeur seuil de confort pour l'intensité lumineuse perçue par le porteur lors de la variation de flux lumineux et, à l'étape b), le niveau de transmission de l'état clair et/ou foncé du filtre est déterminé en fonction de cette valeur seuil de confort (voir exemple 4).

De manière générale, ladite grandeur représentative de la sensibilité dynamique de l'œil du porteur à la variation du flux lumineux est déterminée en tenant compte d'au moins un des paramètres suivants :

- un paramètre relatif aux habitudes d'exposition à la lumière du porteur passées, présentes et/ou futures : par exemple un éclairement moyen initial de l'œil avant variation du flux lumineux, activités pratiquées/métier, saison, situation géographique, lumière artificielle ou naturelle, durée d'exposition...,
- un paramètre relatif à la sensibilité statique au flux lumineux du porteur,
- un paramètre relatif à une amplitude de la variation temporelle et/ou spatiale d'intensité et/ou de spectre du flux lumineux,
- un paramètre subjectif relatif aux performances visuelles du porteur pour des conditions lumineuses et/ou de variation lumineuse données,
- un paramètre subjectif relatif au confort visuel pour des conditions lumineuses et/ou de variations lumineuses données,
- un paramètre lié à l'âge du porteur,
- un paramètre relatif à l'utilisation de lunettes de soleil,
- un paramètre lié à une densité et/ou à une répartition du pigment maculaire de l'œil du porteur,
- un paramètre lié à une capacité d'adaptation de la rétine à la lumière ou à l'obscurité,
- un paramètre lié à une dynamique de réponse pupillaire à la variation lumineuse et/ou à une autre caractéristique pupillaire,
- un paramètre relatif à une pathologie visuelle ou une anomalie oculaire éventuelle du porteur,
- un paramètre lié à un seuil de variation du confort visuel et/ou des performances visuelles exprimé ou mesuré.

De manière générale, les données collectées par un questionnaire ou par des mesures sont transmises à une unité de traitement informatique programmée pour réaliser les étapes d'analyse subséquentes. Cette unité de traitement informatique réalise l'étape b) de détermination du filtre.

Selon une première possibilité, l'unité de traitement information possède une mémoire stockant une liste de différents filtres disponibles. La détermination du filtre est alors réalisée par l'unité de traitement informatique qui choisit le filtre de la liste présentant les caractéristiques les plus proches des caractéristiques optiques déterminées en fonction de la grandeur représentative de la sensibilité dynamique du filtre déterminée à l'étape a).

Tous les types de filtres disponibles avec leurs caractéristiques sont répertoriés. Pour les filtres passifs : les spectres d'absorption et/ ou transmission leur polarisation, la variation spatiale du spectre d'absorption ou de transmission sur la surface du filtre sont précisés.

Pour les filtres passifs interférentiels, la variation du spectre de transmission et/ou de réflexion en fonction de l'angle d'incidence de la lumière sur le filtre est également répertoriée.

Pour les filtres photochromiques : la vitesse de passage d'un état à un autre, le spectre de transmission ou d'absorption ou la transmission moyenne minimale et maximale de chaque état clair ou foncé sont enregistrés.

Pour les filtres actifs: la vitesse de passage d'un état à un autre, le spectre de transmission ou d'absorption ou la transmission moyenne minimale et maximale de chaque état clair ou foncé sont enregistrés ainsi que la présence de capteurs afin de caractériser l'environnement lumineux, de capteurs de gestion du filtre (gestion de la transmission, vitesse, capteurs liés à l'asservissement du filtre, etc...) et caractéristiques de la technologie embarquée (consommation énergie, stabilisation du système, communications d'informations...).

Selon une deuxième possibilité, l'unité de traitement détermine les caractéristiques optiques souhaitées à l'étape b) et commande la fabrication d'un filtre et/ou d'une lentille ophtalmique munie d'un filtre comportant ces caractéristiques optiques précises.

Le filtre pour une lentille ophtalmique destinée à être placée devant l'œil d'un porteur, déterminé grâce au procédé décrit précédemment, améliore ou maintient le confort visuel et/ou les performances visuelles dudit porteur.

Ce filtre appartient à une lentille ophtalmique destinée à être placée devant l'œil du porteur, par exemple dans une monture de lunettes.

Selon une première famille de procédé, la détermination de la grandeur relative à la sensibilité dynamique de l'œil du porteur est réalisée à partir d'une mesure quantitative objective d'une caractéristique physique ou physiologique de cet œil du porteur.

Cette première famille regroupe les exemples 1, 2 et 3.

## EXEMPLE 1

Dans cet exemple, à l'étape a), ladite grandeur relative à la sensibilité dynamique de l'œil du porteur est déterminée en fonction d'une ou plusieurs valeurs mesurées de densité et/ou de répartition du pigment maculaire dans l'œil du porteur.

Le pigment maculaire (PM) est situé dans la zone maculaire de la rétine, sur les 6° centraux d'excentricité rétinienne $\varepsilon$ (Wolf-Schnurrbusch et al., « Ethnic differences in macular pigment density and distribution », Invest. Ophthalmol. Vis. Sci. 2007, 48(8), pp. 3783-3787; Bernstein PS, « The value of measurement of macular carotenoid pigment optical densities and distributions in age-related macular degeneration and other retinal disorders », Vision Res. 2010). Il est composé de lutéine et de zéaxanthine (les caroténoïdes de l'œil). Il est localisé dans la couche plexiforme externe de la rétine et a pour rôle d'absorber le flux lumineux compris dans une gamme spécifique de longueurs d'onde comprises entre 400 et 500 nm, préférentiellement entre 430 et 480 nm. Ce pigment maculaire présente par ailleurs un pic maximum d'absorption d'environ 40% autour d'une longueur d'onde de 460 nm.

Le pigment maculaire a pour fonction de protéger les tissus cellulaires des effets néfastes de la photo-oxydation provoquée par la lumière bleue de longueur d'onde comprises entre 400 et 500 nm, préférentiellement entre 430 et 480 nanomètres et de diminuer la diffusion de la lumière bleue en l'absorbant.

Avec l'âge, la densité de ce pigment maculaire, noté ici diminue de sorte qu'il existe une corrélation forte entre la concentration de ce pigment maculaire et le risque d'apparition d'une dégénérescence maculaire liée à l'âge, ou « DMLA » (voir par exemple Beatty S. et al., Invest. Ophthalmol. Vis. Sci. 2001; 42:439-446).

Le pigment maculaire peut avoir une distribution spatiale différente selon les porteurs. On distingue une répartition en pic ou en forme de tore. La première montre une diminution progressive de la densité du pigment maculaire en fonction de l'excentricité. Il arrive aussi d'observer une cavité centrale dans la distribution spatiale du pigment maculaire au niveau maculaire. On parle alors de répartition en forme de *« donuts »* ou de chapeau mexicain.

Le pigment maculaire a un impact dans les performances visuelles d'un individu : il permet, d'une part, de diminuer l'impact des aberrations chromatiques sur la vision, et, d'autre part, de réduire l'éblouissement.

On notera enfin qu'il existe également une corrélation significative entre la baisse de la densité $d_{PM}$ du pigment maculaire et, d'un côté, la baisse de l'acuité visuelle et de la sensibilité aux contrastes et, de l'autre côté, l'augmentation du temps de récupération des performances visuelles après une augmentation du flux lumineux rapide (Stringham et al., « Macular pigment and visual performance under glare conditions ». Optom. Vis. Sci. 2008, 85(2), pp. 82-88).

On connaît des appareils pour mesurer la densité et la répartition spatiale du pigment maculaire à l'intérieur de l'œil d'un porteur : appareil MPS II (http://www.horus-pharma.com/index.php/fr/hi-tech/mpsii) de la société Horus Pharma, appareil « VisuCam » (http://www.zeiss.com/meditec/en_de/productssolutions/ophthalmology-optometry/retina/diag nostics/fundus-imaging/visucam-500.html) de la société Zeiss.

La littérature (Stringham et al, 2011 : « Macular Pigment and Visual Performance in Glare: Benefits for Photostress Recovery, Disability Glare, and Visual Discomfort » Investigative Ophthalmology & Visual Science Septembre 2011, Vol.52, 7406-7415 et Stringham et al, 2008 : Stringham JM, Hammond BR, « Macular pigment and visual performance under glare conditions », Optom Vis Sci. Février 2008;85(2):82-8) et les études de la demanderesse ont montré un lien entre la densité du pigment maculaire et le seuil de sensibilité statique à la lumière, notamment chez les personnes âgées de plus de 65 ans.

En particulier, plus la densité du pigment est faible, plus le seuil de sensibilité à la lumière est faible, donc plus la photosensibilité est grande.

La densité du pigment est exprimée par une valeur comprise entre 0 et 1, avec la valeur 0 qui correspond à une densité minimale et la valeur 1 qui correspond à une densité maximale. Les densités minimale et maximale sont déterminées statistiquement, par exemple d'après l'ensemble des études menées sur ce sujet.

Le seuil de sensibilité à la lumière correspond à l'intensité lumineuse à partir de laquelle un inconfort visuel est exprimé par le porteur.

Le confort ou l'inconfort visuel du porteur est quantifié par celui-ci à l'aide par exemple d'un indicateur de confort subjectif compris entre 1 et 5 sur une échelle d'évaluation standardisée, comme cela est décrit plus en détails dans l'exemple 5. Ainsi, le niveau de protection du filtre, c'est-à-dire son absorption et/ou sa réflectance, en particulier dans le domaine des longueurs d'onde du bleu entre 400 et 500 nm doit être plus élevé lorsque la densité du pigment maculaire est faible.

Autrement dit, à l'étape b), le filtre déterminé présente une transmission d'autant plus faible que la densité du pigment maculaire est faible.

En outre, un temps de récupération de la vision suite à une variation de flux lumineux impliquant un changement brusque et important de l'intensité de ce flux lumineux, est également lié à la densité de pigment maculaire de l'œil du porteur.

On définit le temps de récupération de la vision comme le temps mis par l'œil pour retrouver un niveau de performances visuelles prédéfinies, soit de manière absolue par un seuil d'acuité ou de mesure de sensibilité aux contrastes par exemple ou de manière relative par un pourcentage de la performance de l'œil avant la variation de flux lumineux.

Le temps de récupération de la vision est ici défini comme le temps mis par l'œil du porteur pour retrouver les performances visuelles initiales qu'il avait avant la variation de flux lumineux.

Plus la densité du pigment maculaire de l'œil du porteur est faible, plus ce temps de récupération après une augmentation brusque et importante de l'intensité est long (Stringham JM, Hammond BR, « The glare hypothesis of macular pigment function », Optom Vis Sci., Septembre 2007, 84(9), 859-64, and « Macular Pigment and Visual Performance in Glare: Benefits for Photostress Recovery, Disability Glare, and Visual Discomfort », Investigative Ophthalmology & Visual Science Septembre 2011, Vol.52, 7406-7415).

De manière similaire, le temps de récupération de la vision en condition d'adaptation à l'obscurité est également corrélé à la valeur de la densité du pigment maculaire. Plus la densité du pigment maculaire est faible, plus le temps de récupération est augmenté en condition d'obscurité (Stringham JM & al., « Macular Pigment and Visual Performance in Low-Light Conditions », Invest Ophthalmol Vis Sci., Avril 2015, 56(4), 2459-68) .

Ainsi, à l'étape a), la grandeur relative à la sensibilité dynamique de l'œil du porteur aux variations de flux lumineux peut être déterminée comme la valeur de la densité de pigment maculaire ou encore comme la valeur du temps de récupération de la vision du porteur après une variation de flux lumineux donnée.

Selon un premier mode de réalisation, à l'étape a), on mesure la densité du pigment maculaire de l'œil du porteur destiné à recevoir le filtre.

La mesure de la concentration du pigment maculaire peut être effectuée grâce à une méthode de mesure objective physique d'autofluorescence telle que celle implémentée dans l'appareil VisuCam de Zeiss ou bien grâce à une méthode subjective dite « photométrie Flicker hétérochromique » (Creuzot-Garcher et al., « Comparison of Two Methods to Measure Macular Pigment Optical Density in Healthy Subjects », Retina 2014 IOVS, May 2014, Vol. 55, No. 5, pp. 2941-2947).

On considère soit la densité moyenne du pigment maculaire (par exemple obtenue par une méthode de type « *Flicker hétérochromique* »), soit l'ensemble de la répartition du pigment maculaire (par exemple obtenue grâce à une méthode par photographie).

En fonction de la densité et de la répartition spatiale du pigment maculaire, on peut déterminer la réponse spectrale du filtre à l'étape b).

En particulier, à l'étape b), on détermine l'absorption du filtre comme étant conforme à la courbe d'absorption du pigment maculaire en fonction de la longueur d'onde, c'est-à-dire identique à cette courbe, mais de densité variable selon la densité du pigment maculaire mesurée chez le sujet.

De manière préférentielle, on détermine le filtre de sorte que le système formé par le filtre et l'œil du porteur présente une transmission proche de la transmission d'un œil de référence. Par « œil de référence », on entend un œil humain dont les photorécepteurs présentent une sensibilité moyenne. Par « proche » on entend que la transmission spectrale du système formé par le filtre et l'œil du porteur est comprise dans une marge prédéfinie autour de la transmission spectrale de l'œil de référence. Typiquement, cette marge peut être de plus ou moins 15% autour de la transmission spectrale de l'œil de référence.

En d'autres termes, le spectre du filtre déterminé est mimétique à celui du spectre du pigment maculaire.

La transmission du filtre est déterminée en fonction de la valeur de la densité du pigment maculaire.

En effet, la valeur de la densité du pigment maculaire indique le degré de protection à rehausser pour préserver la rétine.

Plus précisément, à l'étape b), pour une densité du pigment maculaire inférieure à 0,2, le filtre doit compenser fortement le rôle protecteur du pigment maculaire. Le taux d'absorption $A(\lambda)$ du filtre est déterminé pour être identique à celui du pigment maculaire avec un taux d'absorption maximum de 40 % pour une longueur d'onde de 460 nm.

Pour une densité du pigment maculaire comprise entre 0,2 et 0,6, le filtre doit suppléer une partie des fonctions du pigment maculaire car la densité de celui-ci n'est pas optimale. La transmission du filtre est déterminée pour compenser le manque d'absorption du pigment maculaire en proportion de la perte : le taux d'absorption $A(\lambda)$ du filtre est alors défini par la relation $A(\lambda) = (1-d) \times f(\lambda)$, où d représente la densité du pigment maculaire mesuré à l'étape a) et $f(\lambda)$ est le taux d'absorption du pigment maculaire à la longueur d'onde $\lambda$.

Pour une densité du pigment maculaire supérieure à 0,6, le filtre a alors un rôle de prévention (par exemple de la DMLA).

Le filtre est alors déterminé pour renforcer l'action du pigment maculaire : le taux d'absorption $A(\lambda)$ du filtre est également défini par la relation : $A(\lambda) = (1-d) \times f(\lambda)$, où d représente la densité du pigment maculaire mesuré lors de la première opération et $f(\lambda)$ est le taux d'absorption du pigment maculaire à la longueur d'onde $\lambda$.

Afin d'adapter le filtre et d'optimiser le spectre du filtre à déterminer à l'étape b), il est également possible de tenir compte de la répartition rétinienne du pigment maculaire et des caractéristiques spectrales du flux lumineux caractéristique.

Par exemple, on peut prévoir d'augmenter le taux d'absorption du filtre d'une quantité fonction de la densité moyenne du pigment maculaire et/ou en fonction de la répartition rétinienne de ce pigment (cf. Wolf-Schnurrbusch *et al., op. cit.*).

La répartition du pigment maculaire ne suit pas toujours une fonction gaussienne, centrée sur la fovéa. Elle peut suivre une forme différente, en forme dite « de chapeaux mexicains » ou de « donut ». Le filtre doit tenir compte de la répartition de ce pigment maculaire pour le compléter au mieux.

On peut prévoir également que le filtre présente un taux d'absorption non uniforme sur sa surface de manière à s'adapter à la répartition spatiale du pigment maculaire.

De manière avantageuse, le filtre sera un filtre adaptatif dont le taux d'absorption n'est pas uniforme et est ajusté en temps réel sur sa surface, par exemple asservi par un dispositif de suivi de regard.

Il est également possible d'adapter le taux d'absorption du filtre au contenu spectral du flux lumineux caractéristique. Cette adaptation peut être statique ou dynamique.

Selon un deuxième mode de réalisation, à l'étape a), on mesure la densité du pigment maculaire de l'œil du porteur destiné à recevoir le filtre et on en déduit un temps de récupération de la vision du porteur pour une variation de flux lumineux donnée par estimation, ou on mesure directement ledit temps de récupération de la vision du porteur.

Dans le premier cas, l'estimation du temps de récupération est par exemple réalisée en fonction d'une base de données prédéterminées regroupant les valeurs de ce temps de récupération et les valeurs de densité du pigment maculaire mesurées pour différents porteurs.

Le temps de récupération de la vision du porteur peut être déterminé expérimentalement par un test de sensibilité adaptométrique qui sera décrit en détails plus loin (voir l'es exemples 2 et 4 pour une variation négative de l'éclairement, des tests similaires peuvent être envisagés pour une variation positive de l'éclairement .

A l'étape b), le filtre est déterminé pour améliorer les capacités de récupération du porteur après une telle variation de flux lumineux, c'est-à-dire pour diminuer le temps de récupération de la vision du porteur.

A cet effet, à l'étape b), il est prévu de tester, sur le porteur, différents filtres présentant des spectres de transmission différents, par exemple ayant une transmission faible pour un domaine de longueurs d'onde donné différent.

Ce domaine de longueurs d'onde par exemple centré sur les longueurs d'onde d'absorption maximale du pigment maculaire.

Ensuite, à l'étape b), on évalue le temps de récupération de la vision du porteur après une variation de flux lumineux donnée, pour chaque filtre testé, grâce audit test de sensibilité adaptométrique.

On détermine le filtre en choisissant l'un des filtres testés pour lequel le temps de récupération mesuré à l'étape b) est inférieur au temps de récupération déterminé à l'étape a), ou en déterminant les caractéristiques du filtre choisi en fonction des caractéristiques des filtres testés pour lesquels le temps de récupération mesuré à l'étape b) est inférieur au temps de récupération déterminé à l'étape a).

Ainsi, avec le filtre, le porteur perdra moins en performance visuelle et optimisera son confort visuel lors des variations de flux lumineux.

## EXEMPLE 2

Dans cet exemple, on décrira un procédé de détermination d'un filtre selon l'invention selon lequel, à l'étape a), ladite grandeur relative à la sensibilité dynamique de l'œil du porteur est déterminée en fonction d'une ou plusieurs valeurs mesurées d'un temps de récupération de la vision du porteur après une variation négative de l'intensité du flux lumineux. On effectue à cet effet le test de sensibilité adaptométrique.

Il est alors prévu, à l'étape a) d'effectuer les sous-étapes suivantes :

a1) une étape de soumission du porteur à ladite variation de flux lumineux, et
a2) une étape de mesure d'une grandeur relative à l'adaptation de l'œil à cette variation de flux lumineux, réalisée sur le porteur soumis à ladite variation de flux lumineux.

A l'étape a1), on soumet le porteur à un flux lumineux d'intensité prédéterminée non nulle pendant une première phase d'exposition, puis on soumet le porteur à un flux lumineux d'intensité inférieure, par exemple proche de zéro (obscurité).

Ce passage d'un flux lumineux important à un flux lumineux plus faible peut par exemple simuler le passage d'un environnement à forte luminosité en extérieur à un environnement beaucoup plus sombre, en intérieur ou dans un tunnel.

A l'étape a2), on mesure une grandeur caractéristique des performances visuelles de l'œil ou des yeux du porteur.

Plus précisément, à l'étape a2), on mesure une grandeur caractéristique des performances visuelles de l'œil ou des yeux du porteur moyenne pendant une période de temps déterminée après le début de la deuxième phase et/ou un temps d'adaptation à l'obscurité correspondant au temps de récupération de la vision du porteur nécessaire pour que ladite grandeur caractéristique de ses performances visuelles retrouve une valeur prédéterminée.

Cette valeur prédéterminée est de préférence prédéterminée en fonction de sa valeur initiale avant la variation du flux lumineux.

Ensuite, on trace l'évolution temporelle d'un paramètre représentant la sensibilité rétinienne absolue du porteur, via un test automatisé au cours duquel un stimulus lumineux de faible luminance initiale voit sa luminance diminuer au fur et à mesure que la rétine s'adapte. Le programme automatisé utilise une stratégie en escalier pour suivre l'évolution du seuil de sensibilité pendant l'adaptation à l'obscurité des yeux.

Des tests de faux positifs sont inclus aléatoirement au cours du test et leurs résultats donnent une indication sur la fiabilité de la mesure.

Plus précisément, ici, lors de l'étape a1), le porteur est soumis à un flux lumineux visible blanc, par exemple un spectre de diode électroluminescente LED blanc neutre, produisant un éclairement au niveau de l'œil du porteur compris entre 500 et 1000 lux ou une luminance comprise entre 100 et 300 Cd/m$^2$ pendant 5 minutes. La lumière est distribuée en condition Ganzfeld, c'est-à-dire en champ total. Pendant cette première phase, le porteur fixe un point central de son champ de vision.

En variante, lors de l'étape a1), le porteur est soumis à un flash lumineux dont la durée est inférieure ou égale à 1 seconde.

De manière générale, la luminance, le spectre et la durée du stimulus lumineux lors de l'étape a1) sont adaptables, afin notamment de se rapprocher au mieux de conditions d'exposition lumineuse réalistes.

Puis, le porteur est soumis à une phase d'adaptation à l'obscurité pendant 10 à 30 minutes.

Pendant cette deuxième phase, il est demandé au porteur d'appuyer sur une poire ou un bouton dès qu'il perçoit un stimulus lumineux circulaire de 10° d'étendue angulaire présenté au centre d'un écran ou d'un dôme placé devant le porteur, apparaissant pendant 100 à 300 millisecondes et toutes les 3 secondes.

La luminance du stimulus varie entre 30 dB correspondant à 0,318 cd/m$^2$ et 80 dB, correspondant à 0,318.10^-5 cd/m$^2$ par pas de 1 dB. Le niveau 0 dB est fixé à 318 cd/m$^2$ (référence du périmètre Goldmann). La luminance est ici exprimée en dB par rapport à cette valeur de référence, 0,318 cd/m$^2$ correspondant alors à 10*log(0,318/318) = 30 dB.

Lorsque le porteur perçoit le stimulus, la luminance du stimulus diminue par pas de 1 dB.

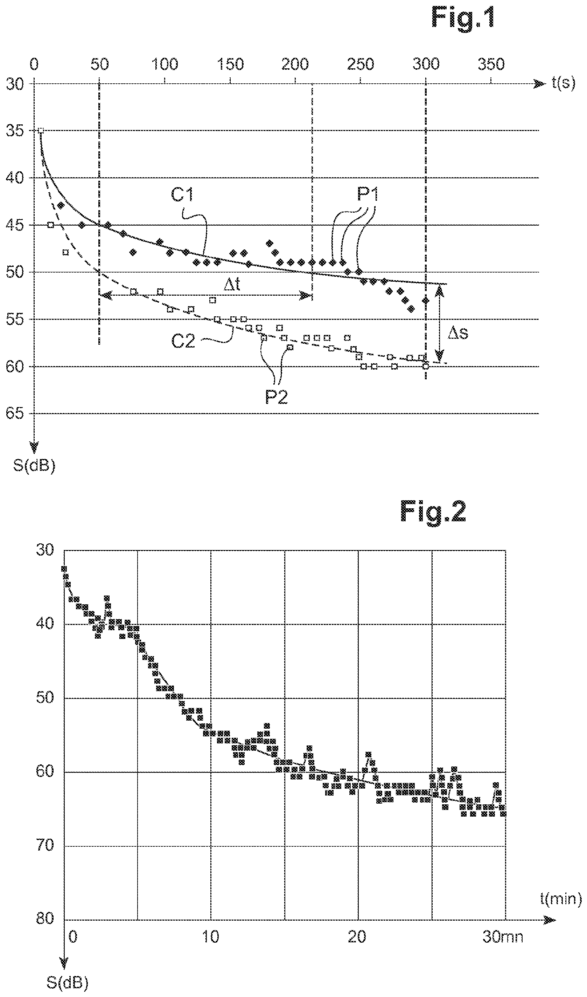

La valeur de luminance en dB détectée par le porteur à un instant donné du test constitue la valeur de sensibilité du porteur à cet instant. Elle est reportée sur la figure 1 de manière à tracer l'évolution de cette sensibilité dans le temps.

Si le porteur ne perçoit pas le stimulus, et donc n'appuie pas sur la poire ou le bouton pendant le temps imparti, la luminance du stimulus remonte légèrement. Pendant toute la durée de la phase d'adaptation à l'obscurité, les yeux du patient sont contrôlés par une caméra infrarouge, afin de s'assurer qu'il ne s'endort pas ou qu'il ne garde pas la fixation centrale sur le stimulus.

A la fin de l'étape a2), l'évolution temporelle de la sensibilité S en dB détectée par le porteur est tracée. Cette courbe d'adaptométrie, représentée sur les figures 1 et 2, comporte deux phases, dont seule la première est représentée sur la figure 1.

La première phase précoce correspond à l'activité des photorécepteurs cônes, impliqués dans la vision diurne. Cette phase dure moins de 6 minutes et est généralement fixée à 5 minutes dans la littérature, voir par exemple "Comparison of AdaptRx and Goldmann-Weekers Dark Adaptometers", John G. Edwards1, David A. Quillen, M.D.2, Laura Walter2, D. Alfred Owens, Ph.D.3 and Gregory R. Jackson, Ph.D.2 ; "A short-duration dark adaptation protocol for assessment of age-related maculopathy", Gregory R. Jackson & John G. Edwards, J ocul biol dis inform (2008) 1:7-11 ; "Measurement Error of the AdaptRx Dark Adaptometer for Healthy Adults and AMD Patients", Laura E. Walter, C.O.A. 1, David A. Quillen, M.D.1, John G. Edwards, M.S., M.B.A.2, D. Alfred Owens, Ph.D. 3 & Gregory R. Jackson, Ph.D.1. Cette première phase suit une évolution logarithmique.

Elle est suivie d'une phase plus lente, visible sur la figure 2, qui atteint un seuil nettement plus bas et qui est due aux bâtonnets.

Sur la figure 2, la première phase correspond à la courbe enregistrée entre 0 et 5 minutes environ tandis que la deuxième phase correspond à la courbe enregistrée entre 5 et 30 minutes. La courbe représentée sur cette figure est ici obtenue avec l'appareil d'adaptométrie commercial MonPack ONE de Metrovision.

L'analyse de la sensibilité adaptométrique des cônes sur les 5 premières minutes de phase à l'obscurité est un très bon indicateur de la sensibilité des yeux du porteur à une diminution importante de flux lumineux au cours du temps.

L'analyse consiste à calculer l'aire sous la courbe de la sensibilité en dB jusqu'à 5 minutes (première phase de la courbe d'adaptométrie) afin de définir la sensibilité intégrée de l'œil du porteur pendant ces 5 premières minutes (= 300 s) après le passage à l'obscurité (en dB).

La figure 1 montre l'évolution temporelle de la sensibilité en dB de l'œil du porteur en fonction du temps écoulé après le passage à l'obscurité sur les cinq premières minutes, c'est-à-dire 300 secondes.

Sur cette figure, deux sets de données de sensibilité sont représentés : un premier set correspond aux points de forme losange P1 mesurés pour le porteur muni d'un filtre de référence dont la transmission visuelle sur le visible entre 380 et 780 nm est égale à 98%, équivalent à une absence de filtre.

Le deuxième set de données correspond aux points P2 de forme carré, mesurés pour le porteur muni d'un filtre T1. Ce filtre T1 correspond à l'état clair d'un filtre présentant des propriétés photochromiques. Dans cet état clair, le filtre T1 bloque 40% de la lumière bleu-violet entre 400 et 455 nm et laisse passer les autres longueurs d'onde du visible.

Sa transmission est donc de 50% entre 400 et 455 nm.

Sa transmission visuelle sur tout le visible est comprise entre 85 et 90% entre 380-780 nm puisque le filtrage est sélectif.

On définit ici la transmission visuelle du filtre comme la transmission du filtre optique pondérée par l'illuminant solaire de référence D65 et la sensibilité photopique de l'œil (ISO 13666: 1998 Standard- ISO 8980-3 Standard)

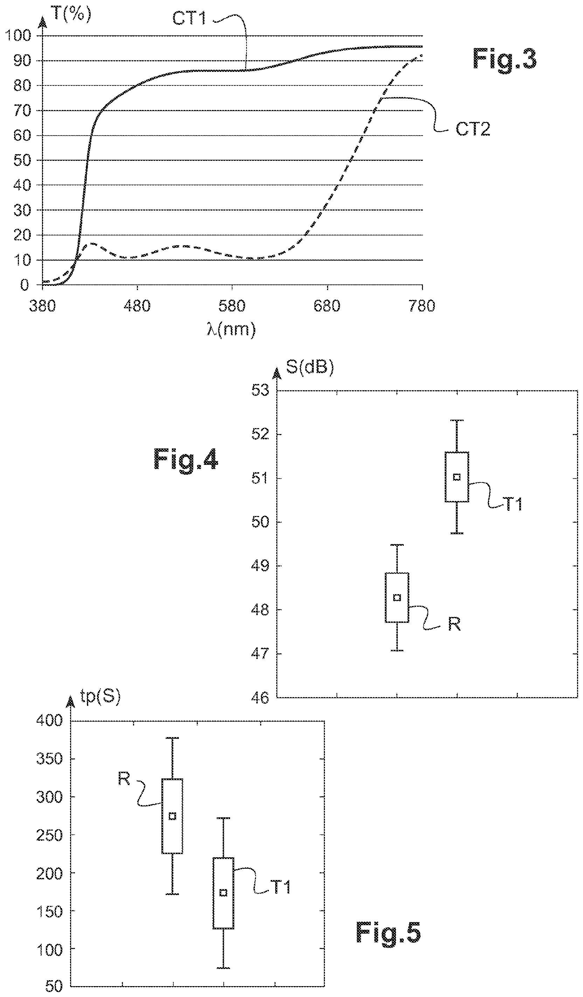

La transmission de ce filtre en fonction de la longueur d'onde est représentée ici sur la figure 3. Sur cette figure, la courbe CT1 correspond à la transmission en fonction de la longueur d'onde de l'état clair du filtre, et la courbe CT2 correspond à la transmission en fonction de la longueur d'onde de l'état sombre de ce filtre. Cette dernière courbe est donnée à titre d'exemple.

Une régression logarithmique de chaque set de données donne les courbes C1 et C2 de la figure 1.

La courbe C1 de formule mathématique S = 3,4118Ln(t) + 31,748 correspond aux mesures avec le filtre de référence, avec un coefficient de corrélation $R^2=0,8951$.

La courbe C2 de formule mathématique S = 5,2267Ln(t) + 29,459 correspond aux mesures avec le filtre T1, avec un coefficient de corrélation $R^2=0,9679$.

A partir de ces courbes, il est possible de déduire le temps de récupération de la vision du porteur muni de chaque filtre, défini ici comme le temps nécessaire aux yeux du porteur pour retrouver une sensibilité égale à 50 dB, correspondant à la détection d'un stimulus lumineux de $0.318.10^{-2}$ cd/m$^2$.

Ici, ce temps de récupération est égal à 210 secondes avec le filtre de référence, et seulement 51 secondes avec le filtre T1 (figure 1).

On observe ainsi une amélioration du temps de récupération $\Delta t$ de 159 secondes, soit un bénéfice de 76% par rapport au filtre de référence clair (équivalent à une absence de filtre).

En outre, 300 secondes ou 5 minutes après le passage à l'obscurité, la sensibilité des yeux du porteur avec le filtre T1 est supérieure de $\Delta S=8$ dB à la sensibilité des yeux du porteur avec le filtre de référence (figure 1).

De manière générale, à l'étape b), plus la sensibilité en dB est faible 5 minutes après le passage à l'obscurité ou plus le temps de récupération des yeux pour retrouver une sensibilité égale à 50 dB est long, plus le filtre déterminé devra être filtrant, soit en transmission visible globale, soit dans la zone spectrale du bleu.

En d'autre terme, la transmission du filtre, soit moyennée sur tout le spectre visible (entre 380 et 780 nm), soit moyennée sur la zone spectrale du bleu-violet (entre 400 et 455 nm) est d'autant plus faible que la sensibilité en dB est faible 5 minutes après le passage à l'obscurité ou que le temps de récupération des yeux pour retrouver une sensibilité égale à 50 dB est long.

Cet exemple est particulièrement adapté aux porteurs pseudophaques munis d'un cristallin artificiel blanc, après une opération de la cataracte.

Ces porteurs présentent en effet un éblouissement d'inconfort significativement plus élevé que les porteurs non pseudophaques. Des travaux de la demanderesse ont en particulier montré que le seuil de photosensibilté de porteurs pseudophaques photosensibles est en moyenne 5 fois plus bas que le seuil de photosensibilité de sujets sains âgés appariés en âge et photosensibles, le seuil étant déterminé dans les mêmes conditions dans les deux cas. Le seuil de photosensibilité correspond à la valeur d'éclairement ou d'intensité du flux lumineux maximale qu'ils peuvent tolérer. En plus d'avoir un seuil de photosensibilité significativement plus bas que les sujets non pseudophaques, les sujets pseudophaques présentent un seuil de première gêne à la lumière, c'est-à-dire correspondant à la valeur d'éclairement ou d'intensité du flux lumineux maximale qu'ils peuvent recevoir sans inconfort, inférieur.

Ils présentent également un temps de récupération plus long lors des variations de flux lumineux.

En particulier, les sujets pseudophaques sont très sensibles aux courtes longueurs d'onde du visible, le bleu-violet, car celles-ci sont beaucoup plus largement transmises jusqu'à la rétine par le cristallin artificiel que par le cristallin d'origine qui filtrait une bonne partie du bleu-violet.

Pour ces porteurs, un filtre bloquant les longueurs d'onde du bleu-violet entre 400 et 455 nm, a minima 20%, préférentiellement 40 à 50% du flux lumineux à ces longueurs d'onde, associé éventuellement à un filtre photochromique pour limiter l'inconfort lumineux dans les environnements extérieurs fortement éclairés sera déterminé. La transmission de ce filtre pour les longueurs d'onde comprise entre 400 et 455 nm est donc de préférence inférieure à 80%, de préférence inférieure à 60%, de préférence inférieure à 50%.

Un exemple de filtre convenant à ces porteurs est par exemple une lentille ophtalmique présentant des propriétés photochromiques ou électrochromiques, avec une transmission de 55% des longueurs d'onde comprises entre 400 et 455 nm à l'état clair (équivalent au filtre T1 mentionné précédemment) et une transmission de 10% de ces longueurs d'onde à l'état foncé (correspondant à un filtre T2, présentant par exemple une transmission similaire à celle de la courbe CT2 de la figure 3 .

Une étude menée par la demanderesse sur 16 porteurs pseudophaques munis de cette lentille ophtalmique a montré une meilleure adaptation à l'obscurité des porteurs munis de celle-ci par rapport à leur adaptation à l'obscurité sans la lentille ophtalmique (cas équivalent à la présence du filtre de référence R mentionné précédemment).

En particulier, une diminution significative du temps de récupération, i.e. une récupération plus rapide à l'éblouissement avec la lentille ophtalmique a été démontrée, avec une diminution de ce temps de plus de 90 secondes.

Plus précisément, la sensibilité brute à 5 minutes est en moyenne de 48 dB sans la lentille ophtalmique avec un intervalle de confiance à 95% compris entre 47 et 49 dB contre 51 dB avec cette lentille avec un intervalle de confiance à 95% compris entre 50 et 52 dB.

Avec la lentille à l'état T1, on obtient un écart de sensibilité $\Delta S$ moyen de + 2 dB par rapport à l'absence de lentille, soit un gain moyen de sensibilité des cônes au bout de 5 min de 6%.

Ces résultats sont résumés dans le tableau 1 suivant, où la colonne R correspond au cas de référence sans la lentille ophtalmique et la colonne T1 correspond au cas où le porteur est muni de la lentille ophtalmique.

| Sensibilité brute à 5 min (dB) | R | T1 |
|---|---|---|
| N | 16 | 16 |
| Moyenne | 48 | 51 |
| Médiane | 48 | 51 |
| Ecart type | 2,27 | 2,39 |
| Min / Max | 42 / 51 | 45 / 56 |
| 1er Quart. / 3ème Quart. | 47 / 50 | 50 / 52 |
| IC -95% / IC +95% | 47 / 49 | 50 / 52 |

Ils sont également représentés graphiquement sur la figure 4, sur laquelle le point carré correspond à la valeur moyenne, le rectangle l'entourant s'étend entre la valeur moyenne plus ou moins l'écart type, et les barres s'étendent entre la valeur moyenne plus ou moins l'intervalle de confiance à 95%.

Le temps de récupération permettant de retrouver une sensibilité de 50 dB est en moyenne de 274 secondes sans la lentille ophtalmique avec un intervalle de confiance (IC) à 95% compris entre 171 et 376 secondes contre 173 secondes avec cette lentille avec un IC à 95% compris entre 74 et 271 secondes. Avec la lentille à l'état T1, on obtient une diminution du temps de récupération moyenne de 101 s par rapport à la situation sans lentille, soit un gain moyen de temps de récupération de la sensibilité à 50 dB de 37%.

Ces résultats sont résumés dans le tableau 2 suivant, où la colonne R correspond au cas de référence sans la lentille ophtalmique et la colonne T1 correspond au cas où le porteur est muni de la lentille ophtalmique.

Tableau 2

| Temps à S=50 dB | R | T1 |
|---|---|---|
| N | 16 | 16 |
| Moyenne | 274 | 173 |
| Médiane | 204 | 122 |
| Min / Max | 105 / 782 | 57 / 829 |
| 1er Quart. / 3ème Quart. | 143 / 351 | 91 / 164 |
| IC -95% / IC +95% | 171 / 376 | 74 / 271 |

Ils sont également représentés graphiquement sur la figure 5, sur laquelle le point carré correspond à la valeur moyenne, le rectangle l'entourant s'étend entre la valeur moyenne plus ou moins l'écart type, et les barres s'étendent entre la valeur moyenne plus ou moins l'intervalle de confiance à 95%.

De manière générale, si le porteur est pseudophaque, se plaint de photosensibilité accrue depuis l'opération et que sa sensibilité dynamique à l'obscurité est faible, on lui propose un filtre photochromique bloquant les longueurs d'onde du bleu-violet. Les caractéristiques photochromiques du filtre sont déterminées pour avoir le meilleur compromis entre transmission et temps de passage vers l'état clair. Plusieurs filtres peuvent être comparés entre eux lors du protocole décrit précédemment afin de déterminer la transmission adaptée et les caractéristiques photochromiques, en particulier en fonction du profil d'exposition lumineuse du porteur.

## EXEMPLE 3

Dans cet exemple, on décrira un procédé de détermination d'un filtre selon lequel, à l'étape a), ladite grandeur relative à la sensibilité dynamique de l'œil du porteur est relative à une caractéristique dynamique de la pupille de l'œil du porteur.

Elle est par exemple déterminée en fonction d'une ou plusieurs valeurs mesurées de cette caractéristique dynamique de la pupille de l'œil du porteur.

Il est alors prévu, à l'étape a) d'effectuer les sous-étapes a1) et a2) mentionnées précédemment.

A l'étape a1), on soumet le porteur à un flux lumineux d'intensité prédéterminé pouvant être nul (condition d'obscurité) ou non nul pendant une première phase d'exposition (ou d'obscurité), puis on soumet le porteur à un flux lumineux d'intensité différente, notamment d'intensité plus élevé.

A l'étape a2), on mesure une grandeur caractéristique des performances visuelles de l'œil ou des yeux du porteur.

La caractéristique dynamique de la pupille est notamment relative à sa taille, par exemple son diamètre, et plus précisément à la variation de cette taille au cours du temps et selon la variation de flux lumineux.

Plus précisément, à l'étape a2), on détermine la variation de taille de la pupille au cours du temps pendant ladite variation de flux lumineux de l'étape a1).

A cet effet, on acquiert des images de l'œil du porteur pendant la variation de flux lumineux et après celle-ci grâce à une caméra à haute fréquence d'acquisition. Cette fréquence d'acquisition est préférence supérieure à 100 Hertz.

L'objectif est ici d'évaluer la dynamique de la pupille face à la dynamique du flux lumineux qui peut varier en intensité, spectre, géométrie de la source, caractéristiques temporelles (flash & continu).

En effet, la pupille se contracte ou se dilate en fonction de l'intensité du flux lumineux incident sur l'œil du porteur. La taille de la pupille varie donc en réponse à la variation de flux lumineux.

La grandeur caractéristique de la pupille peut notamment être relative à un temps de latence de la pupille, c'est-à-dire au temps mis par la pupille pour changer de taille en réponse à la variation du flux lumineux.

Cela correspond à un temps d'adaptation à l'obscurité et à la lumière des yeux du porteur.

Selon la longueur d'onde du flux lumineux, l'intensité des variations temporelles ou les variations spatiales du flux lumineux, la taille de la pupille n'aura pas la même évolution dans le temps.

Les travaux de la demanderesse, détaillés ci-après, ont montré que plus le signal lumineux est transmis rapidement à travers la rétine et jusqu'au muscle sphincter de l'iris, c'est-à-dire plus le temps de latence est court, plus l'inconfort du porteur est importante, et ce, quel que soit l'âge, la luminance, les caractéristiques spectrales et temporelles de la stimulation.

Egalement, les caractéristiques de la variation de flux lumineux sont déterminantes dans la sensation de confort du porteur et dans l'évolution temporelle de son diamètre pupillaire.

Par exemple, dans le cas de sujets jeunes de moins de 40 ans, l'amplitude de constriction pupillaire et/ou le maintien de cette constriction après stimulation lumineuse sont plus importantes dans le cas d'une stimulation lumineuse de longueur d'onde 465 nm que pour une longueur d'onde de 619 nm, quelle que soit la luminance photopique et la durée de ladite stimulation. En effet, dans des conditions lumineuses photopiques, les longueurs d'onde les moins énergétiques du bleu entre 460 et 510 nm activent les cellules ganglionnaires à mélanopsine qui jouent un rôle déterminant dans le maintien de la constriction pupillaire. (Gamlin, Mc Dougal et al., 2007, Human and macaque pupil responses driven by melanopsin-containing retinal ganglion cells, Vision Research, 47(7): 946-954).

Dans un autre exemple, pour une stimulation lumineuse de longueur d'onde 465 nm et à durée de stimulation égale, plus la luminance est grande, plus le maintien de la constriction est important, puisque un nombre croissant cellules ganglionnaires à mélanopsine (sensibles à ces longueurs d'onde du bleu) sont activées.

Dans un autre exemple, à luminance photopique fixe (par exemple entre 100 et 400 Cd/m$^2$) et pour une longueur d'onde d'excitation lumineuse de 465 nm, une augmentation du maintien de la constriction pupillaire est observée pour des temps de stimulation croissants entre 1 ms et 500 ms. Au-delà de 500 ms et jusqu'à par exemple 1 s, le maintien de la constriction pupillaire n'augmente plus. On peut avantageusement utiliser ce résultat pour ajuster la durée de la stimulation lumineuse, en fonction de la caractéristique de la pupille utilisée.

Ainsi pour chaque porteur, il est possible de déterminer la transmission du filtre à déterminer à l'étape b) en fonction du temps de latence mesuré de la pupille du porteur.

On définit à cet effet une valeur seuil de référence du temps de latence de la pupille pour un niveau de confort donné à partir de mesures effectuées sur de nombreux porteurs ou pour le porteur en particulier. Cette valeur seuil de référence est utilisée pour déterminer la transmission du filtre de manière à assurer un temps de latence pupillaire du porteur supérieure à la valeur seuil de référence.

Par exemple, la valeur seuil de référence pour le niveau 3 de confort (sur l'échelle allant de 0 à 5) est égale à 300 ms. Si avec un filtre dont la transmission est de 30%, suite à une variation de flux lumineux, le temps de latence de la pupille du porteur est mesuré comme étant égal à 220 ms, cela signifie que ce filtre ne protège pas suffisamment ce porteur des variations de flux lumineux.

La transmission moyenne du filtre est alors diminuée de manière à ce que le temps de latence de la pupille du porteur déterminé à l'étape a) devienne supérieur ou égale à la valeur seuil de référence. Les caractéristiques spectrales du filtre, c'est-à-dire sa transmission pour différentes plages de longueurs d'onde peut également être optimisée pour allonger le temps de latence de la pupille.

Selon le procédé décrit dans cet exemple 3, le filtre est alors déterminé en fonction du temps de latence de la pupille de l'œil du porteur mesuré et de la valeur seuil de ce temps prédéterminée correspondant à un niveau de confort visuel donné. On peut envisager en variante que le filtre soit déterminé en fonction d'autres caractéristiques dynamiques de la pupille de l'œil, telle la vitesse de temps de recouvrement de la pupille suite à la stimulation ou l'amplitude de constriction.

La détermination de la valeur seuil de référence du temps de latence peut être déterminée de la manière suivante lors d'une étape préalable d'étalonnage.

Il s'agit d'établir une relation de corrélation entre le temps de latence mesuré et le niveau de confort du porteur.

Pour chaque porteur d'un groupe de porteur comprenant un grand nombre de porteurs, par exemple au moins 10 porteurs, on réalise les étapes a1) et a2) avec différentes variations du flux lumineux. A l'étape a2), on collecte en outre une information relative au confort visuel du porteur à la suite de la variation de flux lumineux de l'étape a1). Par exemple, on demande au porteur de noter le niveau de confort ressenti par l'indicateur de confort déjà mentionné précédemment.

Pour ces différentes conditions, on demande à la personne de noter le confort entre 0 et 5 selon l'échelle décrite dans l'exemple 5. Puis, une analyse statistique permet de déterminer la relation de corrélation entre niveau de confort et temps de latence.

Plus précisément, on réalise les mesures suivant le protocole suivant :
La salle de mesure est éclairée par un flux lumineux initial non nul. Le porteur est équipé des lunettes d'essai grand champ avec l'addition minimale déterminée pour que ce porteur puisse percevoir une cible lumineuse nette à 33 cm.

Le porteur est installé sur une chaise et pose son menton sur une mentonnière dédiée. Une coupole qui émet une lumière diffuse homogène est placée devant lui. La coupole est éteinte. On éteint la lumière de la salle de mesure et le porteur est placé dans l'obscurité pendant au moins 1 min, et idéalement entre 10 et 15 minutes. Le porteur a pour consigne de fixer un point lumineux dont la luminance est égale à 1 candela par mètre carré ($cd/m^2$) situé au centre de la coupole et du champ visuel de celui-ci.

La coupole émet des stimulations : A chaque stimulation, elle s'allume une seconde toutes les dix secondes, et émet un flux lumineux de longueur d'onde et de luminance déterminés correspondant à un type de stimulation.

La coupole s'allume quatre fois par type de stimulation.

Les luminances desdites stimulations correspondent à des intensités lumineuses scotopiques croissantes, allant du seuil de sensibilité des porteurs correspondant à une luminance de la source d'environ 0,00001 $cd/m^2$, à une luminance de 0,01 $cd/m^2$. Les longueurs d'onde sont successivement 660, 619, 525, 465, 414 nm pour chaque luminance.

Entre chaque stimulation, on demande au porteur de noter son confort à la stimulation lumineuse sur l'échelle de confort à 5 niveaux déjà mentionnée.

Le porteur est également soumis à des stimulations d'intensités photopiques pour 5 longueurs d'onde, de 1 seconde toutes les 20 secondes, de luminance croissante de 1 $cd/m^2$ à 300 $cd/m^2$.

Entre chaque stimulation, on demande au porteur de noter son confort à la stimulation sur l'échelle de confort à 5 niveaux déjà mentionnée.

Lors d'une deuxième séance de mesures, les yeux du porteur sont initialement adaptés à la lumière ambiante de la pièce. La coupole émet une succession de stimulations d'intensités photopiques pour 5 longueurs d'onde, de 1 seconde toutes les 20 secondes, de luminance croissante de 1 $cd/m^2$ à 300 $cd/m^2$ avec un pas de 1 en logarithme de luminance photopique.

Entre chaque stimulation, on demande au porteur de noter son confort à la stimulation lumineuse sur l'échelle de confort à 5 niveaux déjà mentionnée.

Puis, la coupole émet des stimulations achromatiques combinant plusieurs longueurs d'onde, par exemple les 3 longueurs d'onde : 465, 525 et 619 nm avec une luminance totale comprise dans un intervalle allant de 1$cd/m^2$ à 1500 $cd/m^2$.

Les stimulations achromatiques sont par exemple d'une durée d'une seconde toutes les 20 secondes.

En parallèle, pour chaque stimulation le temps de latence de la pupille du porteur est déterminé par analyse d'images de cette pupille enregistrées pendant le test, à haute fréquence.

De manière générale, l'analyse de ces mesures a permis à la Demanderesse de montrer que plus le signal lumineux est transmis rapidement à travers la rétine et jusqu'au muscle sphincter de l'iris, plus la sensation de gêne est importante, et ce, quel que soit l'âge, la luminance, les caractéristiques spectrales et temporelles de la stimulation.

Avantageusement, l'analyse des mesures peut également prendre en compte la présence de différents sous-groupes de porteurs au sein du groupe de porteurs testés.

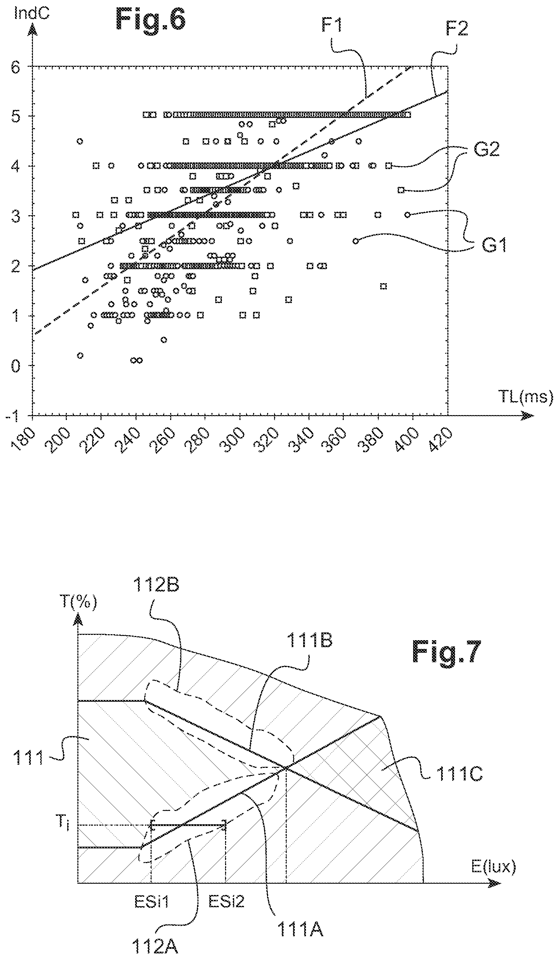

La figure 6 résume les résultats de ces mesures. Les temps de latence TL en millisecondes mesurés en fonction du niveau de l'indicateur de confort IndC correspondant évalué par les porteurs après chaque variation de flux lumineux donnée.

Deux sous-groupes de porteurs sont représentés sur cette figure 6 : une population « jeune », dont l'âge est compris entre 18 ans et 40 ans forme le premier sous-groupe dont les données sont représentées par les points circulaires G1 et une population « sénior », dont l'âge est supérieur à 60 ans, forme le deuxième sous-groupe dont les données sont représentées par les points carré G2.

L'analyse de ces données montre une corrélation entre le temps de latence et l'indicateur de confort, représentée par les courbes de régression linéaire F1 et F2 calculées pour chaque sous-groupe de porteur.

La courbe F1 de formule mathématique IndC = -3,839 + 0,0246*TL correspond au sous-groupe de porteurs « jeunes ».

La courbe F2 de formule mathématique IndC = -0,7665 + 0,0149*TL correspond au sous-groupe de porteurs « seniors ».

Ainsi, il est possible à partir de cette relation de corrélation de déterminer la valeur seuil de référence du temps de latence de la pupille correspondant à n'importe quel niveau de confort donné (ex : 4 ou 3) et valable statistiquement pour un sous-groupe de porteur.

Une valeur seuil de référence peut être déterminée de la même manière pour l'ensemble du groupe de porteur. Cette valeur seuil de référence pour l'ensemble du groupe de porteur peut également être déterminée en fonction des valeurs seuil de référence de chaque sous-groupe, par exemple en prenant la moyenne de ces valeurs.

## **EXEMPLE 4**

Dans cet exemple, à l'étape a), la grandeur représentative de la sensibilité dynamique de l'œil du porteur à la variation de flux lumineux est liée à une sensibilité dynamique à l'éblouissement dudit porteur.

Cela est réalisé via des étapes a1) et a2) mentionnés précédemment.

De manière générale, il est connu que l'éblouissement et le port de filtres, de type solaire par exemple, impactent la vision et le confort visuel d'un porteur de lentilles ophtalmiques munies de tels filtres.

Grâce au procédé de détermination de l'invention, on détermine la réponse spectrale du filtre qui permet d'optimiser la vision et le confort du porteur et ce, quel que soit la variation du flux lumineux caractéristique.

Le procédé permet également la personnalisation de la réponse spectrale du filtre, qu'il soit actif ou passif, en fonction du porteur.

Le procédé proposé ici prend également en compte la réfraction du porteur afin d'avoir la meilleur précision possible dans cette mesure, qui se base et intègre les performances visuelles de ce porteur.

Plus précisément, la détermination de la réponse spectrale du filtre est fondée sur ici l'utilisation d'un « cône de prescription » dynamique.

De manière générale, à l'étape a) ladite grandeur représentative de la sensibilité dynamique de l'œil du porteur est alors relative à ce cône de prescription. A l'étape b), on détermine alors le filtre de telle sorte que le flux lumineux reçue par le porteur à travers ce filtre se situe, dans les conditions de flux et de variation de flux qu'il subit, le plus fréquemment possible à l'intérieur de son cône de prescription.

On va décrire ici brièvement le principe général de cette méthode du cône de prescription avant de décrire plus en détail le procédé de détermination du filtre.

Nous décrirons tout d'abord un « cône de prescription » statique prenant en compte l'éblouissement du porteur par un flux lumineux statique. Nous verrons ensuite comment est modifié ce « cône de prescription » afin de prendre en compte les aspects dynamique du confort et de la performance visuelle liés aux variations de flux lumineux.

La méthode de détermination du « cône de prescription » statique comprend les phases suivantes.

Dans une première phase de la méthode, à l'étape a1), on place le porteur dans un environnement lumineux donné, et on détermine à l'étape a2) la transmission du filtre minimum préservant le confort. Cette transmission peut être moyennée sur un intervalle de longueur d'onde ou dépendre de la longueur d'onde. Il s'agit dans ce dernier cas d'une détermination de la transmission pour une longueur d'onde donnée.

Ceci est illustré par la figure 7, sur laquelle on a représenté la transmission du filtre T en fonction de l'éclairement lumineux E. Deux courbes sont représentées sur cette figure : une première courbe 111A de seuil de confort correspond à une transmission minimale du filtre qui délimite deux zones distinctes : une zone de confort située au dessus de la première courbe 111A pour laquelle le porteur n'est pas gêné dans l'environnement lumineux pour réaliser sa tâche ; et une zone d'inconfort située en dessous de cette première courbe 111A pour laquelle le porteur est gêné.

Dans une deuxième phase de la méthode, on détermine, à l'étape a2), pour le même environnement lumineux de l'étape a1), la transmission du filtre maximum maintenant une performance de vision optimale, par exemple : maintien de l'acuité visuelle ou de la sensibilité aux contrastes.

Ceci est illustré sur la figure 7 par la deuxième courbe 111B de seuil de performance visuel correspondant à la transmission maximale du filtre qui définit deux zones distinctes : une zone de performance visuelle située en dessous de la deuxième courbe et une zone de perte de vision située au dessus de cette deuxième courbe 111B.

Dans une troisième phase, on combine les deux approches précédentes pour déterminer le cône de prescription 111 (voir la figure 7).

Ce cône de prescription correspond à un domaine de transmission du filtre en fonction de l'éclairement lumineux pour lequel la performance visuelle et le confort visuel du porteur sont assurés. Ce cône de prescription permet ainsi de déterminer les caractéristiques optiques du filtre (transmission) qui préservent à la fois la performance visuelle et le confort visuel du porteur pour une large gamme donnée d'environnements lumineux.

La zone 111C de la figure 7 correspond à une zone dans laquelle le porteur expérimente à la fois une perte de performances visuelles et une perte de confort visuel.

Les courbes de seuil de confort et de performance visuelle 111A et 111B peuvent être déterminés selon une méthode descendante ou ascendante. Pour la méthode descendante, le porteur commence avec le verre le plus foncé (pour un domaine de longueurs d'onde donné), et diminue l'absorption /augmente la transmission du flux lumineux pour déterminer les seuils (confort et performance). Le porteur part donc d'un état où la rétine est non saturée.

Pour la méthode ascendante, le porteur commence avec le verre le plus clair (pour un spectre donné ou une longueur d'onde donnée), et augmente la transmission/diminue l'absorption du filtre pour déterminer les seuils (confort et performance). Le porteur part d'un état où il peut être ébloui : la rétine est sursaturée de lumière.

Pour déterminer le « cône de prescription » statique, on place le porteur dans un environnement lumineux de sorte qu'il est soumis à un flux lumineux caractéristique contrôlé et paramétré.

Ce flux lumineux caractéristique est caractérisé par :

- une gamme d'éclairement lumineux, par exemple comprise entre 0 et 20000 lux ;
- une gamme de longueurs d'onde visibles, par exemple comprises entre 400 nm et 680 nm ;
- un éclairage diffus ou localisé, directif ou non directif, défini par exemple par une orientation et un diamètre de source lumineuse.

Par souci de simplification, on ne considérera dans cet exemple que les variations d'éclairement pour expliquer le principe de la mise en œuvre du procédé.

La mesure de la sensibilité de l'œil du porteur peut être réalisée en faisant varier continument l'ensemble des paramètres cités ci-dessus afin de caractériser plus précisément le profil de sensibilité à l'éblouissement du porteur.

Il est aussi possible de répéter cette mesure en étudiant l'effet du spectre du flux lumineux caractéristique sur la sensibilité du porteur à la lumière.

Ensuite, le porteur regarde une cible de taille, forme, luminance, contraste de luminance, et de fréquences spatiales définies préalablement (ou de manière générale toute cible caractérisant une capacité visuelle, comme par exemple une cible colorée).

De préférence, la cible est choisie en fonction de l'activité du porteur, à savoir de l'exigence de vision souhaitée pour la tâche visuelle considérée. Elle peut par exemple être liée aux besoins en termes d'acuité visuelle, de sensibilité aux contrastes, à la précision du rendu des couleurs, etc....

Si cela est nécessaire, le porteur porte une paire de lentilles ophtalmiques permettant de corriger sa réfraction de manière optimale (sphère et cylindre).

Il porte également un filtre test placé devant l'un et/ou l'autre de ses yeux, le taux d'absorption et/ou la réponse spectrale de ce filtre test étant variable.

La mesure de la grandeur représentative de la sensibilité dynamique de l'œil du porteur au flux lumineux est alors réalisée au moyen d'un filtre-test placé devant l'œil porteur, dont on fait varier le taux d'absorption et/ou la réponse spectrale.

S'agissant de performance visuelle, on commence l'étape de mesure avec un filtre-test dont le taux d'absorption est élevé (verre le plus foncé).

En effet, dans le cas d'une mesure d'acuité visuelle ou de contraste, ce filtre-test filtre pénalise la vision : le porteur ne reconnait plus la cible.

On demande alors au porteur de diminuer le taux d'absorption du filtre (ou avec l'aide d'un opérateur) jusqu'à ce qu'il retrouve une perception visuelle satisfaisante. On se situe alors au seuil de performance visuelle (passage du « non-vu » au « vu »). Une méthode psychophysique peut également être utilisée pour définir cette zone. On relève le taux d'absorption du filtre qui détermine ce seuil, délimitant la zone autorisant une performance visuelle non dégradée pour le flux lumineux caractéristique considéré.

Ce test est réitéré pour des éclairements lumineux différents du flux lumineux caractéristique. On obtient ainsi une courbe semblable à la deuxième courbe 111B de la figure 7.

On réalise ensuite la même mesure, non plus avec un test de vision, mais en demandant au porteur la zone à partir de laquelle l'éclairement du flux lumineux caractéristique est gênante ou entraîne un inconfort visuel.

Comme précédemment, on obtient alors une courbe semblable à la première courbe 111A de la figure 7.

On détermine ainsi le cône de prescription 111 correspondant à la zone dans laquelle les performances visuelles sont optimales pour une gamme donnée d'éclairement lumineux du flux lumineux caractéristique et une gamme de taux d'absorption du filtre. On connaît également, grâce à ce cône de prescription, l'effet négatif d'un filtre sur les performances visuelles du porteur.

Ce cône peut aussi être défini en fonction de l'intensité du flux lumineux ou de la luminance de la source.

Dans cette zone 111 de prescription, les caractéristiques optiques du filtre telles que le taux d'absorption ou la réponse spectrale sont alors déterminées de manière à ce que le filtre équilibre le confort et les performances visuelles du porteur.

Il est également possible de répéter ces mesures en soumettant le porteur à un flux lumineux caractéristique caractérisé par différents spectres modifié par le filtre ou par la source lumineuse elle même. De cette façon, on évalue l'influence du spectre du flux lumineux caractéristique sur la sensibilité à la lumière de l'œil du porteur. Ceci permet de guider le choix de la ou des caractéristiques optiques du filtre.

On peut réitérer ces mesures en considérant aussi d'autres critères tels que le confort visuel, la perception des couleurs, la perception des mouvements, etc....

On obtient ainsi une gamme de réponse spectrale permettant de maintenir vision et confort.

Un « cône de prescription » dynamique peut également être déterminé de manière à prendre en compte la sensibilité dynamique de l'œil du porteur aux variations de flux lumineux.

En effet, selon les habitudes d'exposition du porteur, son activité, les conditions lumineuses auquel il est confronté (changement brusque de lumière ou progressive), celui-ci aura besoin d'une protection différente pour se positionner dans sa zone de confort.

Pour chaque courbe seuil de confort ou de performance délimitée, on détermine le delta de luminance toléré par le sujet ou autrement dit, les zones dynamiques de confort et de performances 112A, 112B définies à partir de plusieurs conditions de variation du flux lumineux.

Afin de caractériser la sensibilité dynamique du porteur, c'est-à-dire sa capacité d'adaptation aux variations de flux lumineux, on réalise le cône de prescription avec plusieurs paramètres de variations de flux lumineux.

On soumet le porteur à une variation temporelle du flux lumineux: changements instantanés d'intensité lumineuse, ou flash, en moins de 1 s, progression linéaire de la luminance en lumière continue, dans un temps donné, progression par pas de la luminance, par exemple augmentation de manière discrète de l'éclairement lumineux avec une variation de 20% toutes les 1 seconde. D'autres variations de flux sont possibles, tels par exemple des vitesses de variations de flux de 5% de lux /sec (vitesse lente), 25%/sec (vitesse moyenne) et 100%/sec (vitesse rapide).

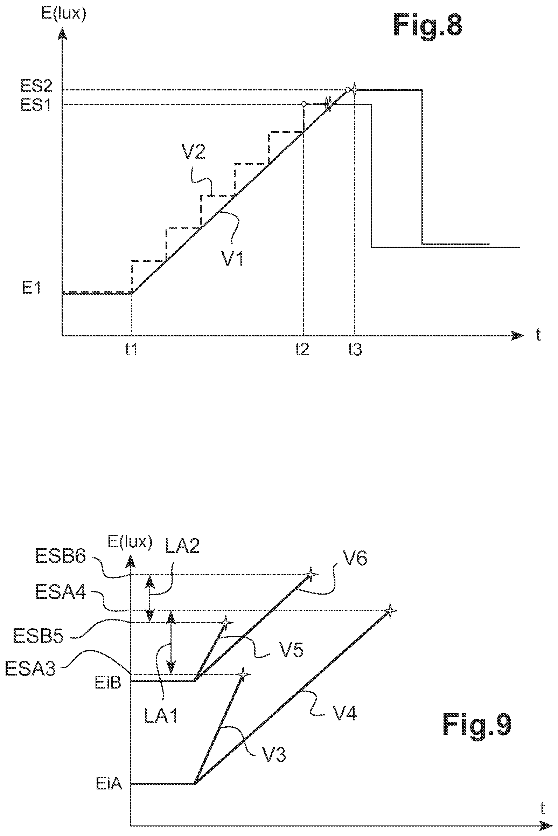

Un changement temporel par pas est ressenti de manière plus agressive par le porteur. Certains porteurs ont des seuils de confort plus important lorsque la variation de luminance est progressive. Le sujet perçoit moins de contraste de luminance. C'est ce qui est représenté sur la figure 8.

Cette figure 8 montre la variation d'éclairement en lux du flux lumineux en fonction du temps. L'éclairement initial de ce flux lumineux est E1. Deux progressions sont représentées : une progression linéaire V1 entre t1 et t3 et une progression pas par pas V2 entre t1 et t2.

La valeur seuil de confort d'éclairement du porteur ES1 pour la progression pas par pas V1 est inférieure à la valeur seuil de confort ES2 pour la progression linéaire V2. Elle est en outre atteinte plus rapidement.

La valeur seuil de confort pour l'éclairement dépend donc du profil temporel de la variation de flux lumineux.

En conséquence, sur la figure 7, à une valeur de transmission Ti donnée du filtre correspond deux valeurs seuil ESi1 et ESi2 d'éclairement pour le seuil de confort du porteur.

Aussi, on peut par exemple déterminer à l'étape b) un filtre électrochromique qui adapte la transmission du filtre pour assurer toujours un changement temporel linéaire de l'éclairement rétinien si ce type de changement optimise les performances visuelles du porteur, c'est-à-dire correspond à une valeur seuil d'éclairement de confort et de performances plus grande que celle obtenue pour des types de changement différents.

La variation temporelle d'intensité du flux lumineux dépend de plusieurs paramètres, notamment de la variation globale d'intensité $\Delta I$, de la durée de cette variation D et de la vitesse de la variation, définie comme la variation globale d'intensité divisée par sa durée.

On peut proposer des gammes de vitesses de variations de flux lente, par exemple 5% de lux /sec, moyenne, par exemple 25%/sec et rapide, par exemple 100%/sec.

En faisant varier des paramètres de flux (vitesse, $\Delta I$ et D), on délimite les zones dynamiques 112A, 112B correspondant à la latitude d'adaptation du porteur aux variations de flux.

Alors, à l'étape b), on peut envisager que la vitesse du changement de la transmission du filtre, c'est-à-dire la vitesse de passage de l'état clair à l'état foncé ou inversement lorsque le filtre est électrochrome, ou à défaut une fonction photochromique du filtre, par exemple la variation globale de transmission entre état clair et foncé et/ou la durée de passage de l'état clair à l'état foncé, soit adaptée de manière à ce que le changement d'éclairement perçu par le porteur pendant la variation d'éclairement du flux lumineux présente des caractéristiques de variation adaptées au porteur.

Plus précisément, à l'étape b), on détermine une durée nécessaire pour passer de l'un à l'autre des états clair et foncé d'autant plus courte que la latitude d'adaptation du porteur est faible.

Ainsi, à l'étape b), on détermine également une variation globale de transmission entre les états clair et foncé du filtre d'autant plus courte de la latitude d'adaptation du porteur est faible.

On peut également déterminer une vitesse seuil de variation du flux lumineux assurant le confort du porteur et déterminer, à l'étape b), la variation globale de transmission du filtre et/ou la durée de passage de l'état clair à l'état foncé pour que la vitesse de variation de flux lumineux perçue par le porteur reste en - dessous de la vitesse seuil déterminée.

Ainsi, par exemple, si le sujet a une vitesse seuil de variation de l'intensité lumineuse, de l'éclairement ou de la luminance égale à une variation de 25% d'augmentation de lux par seconde (25%/sec) et que la variation d'intensité, d'éclairement ou de luminance qu'il subit est de 50%/sec, on déterminera un filtre dont la transmission est de 50%. Si le porteur subit d'autres variations de luminance, une fonction active permettra d'adapter pour chaque situation la transmission du filtre pour atteindre la vitesse seuil de variation d'intensité, de luminance ou d'éclairement de confort cible.

Si le porteur a une capacité d'adaptation à la dynamique du flux lumineux, on obtiendra des écarts entre valeurs extrêmes ESi1, ESi2 des seuils de confort et de performance visuelle grande (figure 7), et donc des zones dynamiques 112A, 112B larges. A l'opposé, si le porteur présente une sensibilité dynamique faible aux variations de flux, les zones dynamiques 112A, 112B seront étroites.

On peut ainsi déterminer un paramètre de latitude d'adaptation du porteur, liée à la largeur des zones dynamiques 112A, 112B.

La détermination du paramètre de latitude d'adaptation du seuil de confort et de performances visuelles va déterminer le besoin de prescription spécifique. Si la valeur de ce paramètre est faible, il sera crucial d'adapter le filtre, en adaptant par exemple sa transmission, pour que le porteur reste dans sa zone de confort dynamique, délimité par le cône de prescription 111 et les zones dynamiques 112A, 112B sur la figure 7, quelque soit l'environnement lumineux auquel il est confronté.

Des filtres photochromiques ou électrochromiques peuvent être à préconiser. La transmission sera choisie pour que le sujet se trouve toujours dans son enveloppe de confort visuel et de performance visuelle pour une intensité donnée et dynamique donnée.

La latitude d'adaptation est dépendante de plusieurs éléments dont entre autre les niveaux d'intensités lumineuses, du spectre de ou des sources lumineuses, de la géométrie de la source lumineuse (taille de la source, rapports des intensités lumineuses entre plusieurs sources,...) et de sa composante temporelle (flash, lumière continue). L'ensemble de ces paramètres peuvent être pris en compte pour caractériser le profil complet de la sensibilité à la lumière du porteur

Par ailleurs, il se peut que le paramètre de latitude d'adaptation dépende de l'état rétinien initial. On prévoit alors de caractériser les zones dynamiques pour différents états rétiniens initiaux, c'est-à-dire pour différentes intensités lumineuses ambiante initiales.

Un exemple est représenté sur la figure 9, qui montre l'évolution temporelle de l'éclairement du flux lumineux en fonction du temps.

Quatre résultats expérimentaux sont ici représenté : on a placé de porteur dans deux flux lumineux d'éclairement initial EiA et EiB différents et pour chaque éclairement initial, on a fait varier cet éclairement avec deux vitesses différentes : les courbes V3 et V5 montrent la variation de l'éclairement avec une première vitesse à partir respectivement des éclairement initiaux EiA et EiB, tandis que les courbes V4 et V6 montrent la variation de l'éclairement avec une deuxième vitesse à partir respectivement des éclairement initiaux EiA et EiB.

On augmente ici l'éclairement jusqu'à ce que le porteur indique un inconfort visuel. La valeur maximale d'éclairement atteinte ESA3, ESA4, ESB5, ESB6 constitue donc la valeur seuil de confort du porteur pour les conditions de variation de flux lumineux correspondantes. On constate que ces valeurs seuil de confort diffèrent selon la valeur initiale de l'éclairement et la vitesse de variation de celui-ci. En outre, le paramètre de latitude d'adaptation, défini ici comme l'écart entre les deux valeurs seuil de confort mesurées pour une même valeur initiale de l'éclairement, est différent selon cette valeur initiale de l'éclairement.

Ainsi, le paramètre de latitude d'adaptation peut constituer la grandeur relative à la sensibilité dynamique du porteur déterminée à l'étape a).

Selon un autre exemple, on réalise à l'étape a) des variations d'éclairement instantanées (brusque).

Pour cela, le porteur est assis devant une coupole à lumière qui émet une lumière diffuse homogène. On soumet le sujet à un éclairement initial donné (20, 200, 2000 et 4000 lux) pendant 90 sec. On applique ensuite un changement brusque d'éclairement positif ou négatif pour arriver à des éclairements finaux de 500, 1000, 2000 et 4000 lux pour une variation positive et de 20, 200, 1000 et 2000 lux pour une variation négative.

Pour chaque situation lumineuse, une valeur de l'indicateur de confort et une grandeur relative à la performance visuelle sont relevés. La grandeur relative à la performance visuelle est par exemple déterminée par un test d'acuité à 10% de contraste.

Par cette analyse, l'évolution de la variation de l'indicateur de confort $\Delta IndC$ selon la variation d'éclairement $\Delta E$ subie par le porteur est déterminée.

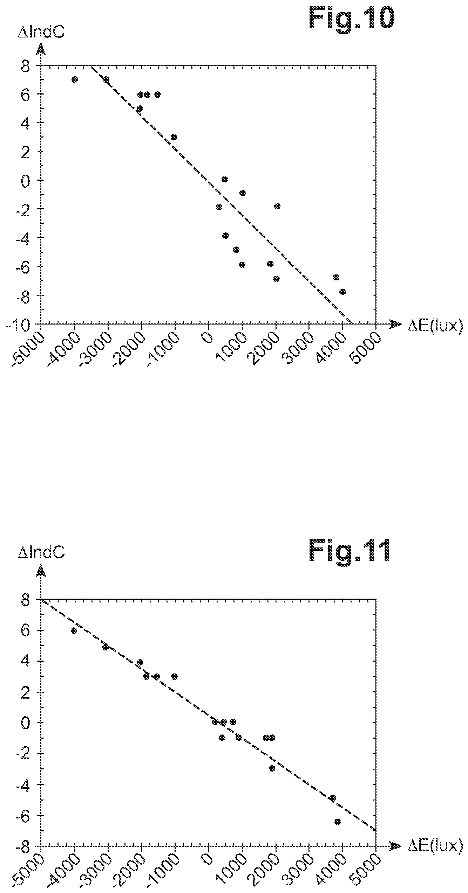

Cette évolution est par exemple représentée par les graphiques des figures 10 et 11 montrant les données enregistrées pour deux porteurs différents.

On peut alors déterminer une variation de l'indicateur de confort maximale autorisée pour le porteur, par exemple de 2 points sur l'échelle d'évaluation du confort. Il est alors possible de déterminer pour chaque sujet, la variation d'éclairement critique à partir de laquelle le sujet sera en inconfort.

La grandeur relative à la sensibilité dynamique des yeux du porteur déterminée à l'étape a) peut alors correspondre à cette variation d'éclairement critique. Elle est déterminée en fonction d'un seuil de variation de confort du porteur, ici de 2 points.

A l'étape b), on détermine alors un équipement solaire actif, comprenant par exemple un filtre électrochrome, pour déterminer en fonction de l'intensité lumineuse de l'environnement du porteur et de l'analyse des variations de luminosité subies grâce à une caméra intégré à cet équipement, un changement de transmission permettant aux yeux du porteur de recevoir en permanence un éclairement et une variation d'éclairement lui permettant de conserver son confort visuel.

Par exemple, le porteur dont les données sont représentées sur la figure 10 se trouve dans un environnement lumineux dont l'éclairement est de 10000 lux et porte un filtre avec une transmission de 50%. L'éclairement reçu par les yeux du porteur est alors de 5000 lux.

Ce porteur va rentrer dans une zone de lumière dont l'éclairement est de 13000 lux.

Le porteur recevra alors 7500 lux si la transmission du filtre est maintenue à 50%. Cela équivaut à une augmentation de l'éclairement de 1500 lux, qui sera associée à une baisse de l'indicateur de confort de 4 points selon les données de la figure 10.

Le filtre proposé à l'étape b) permet alors de limiter la variation d'éclairement à 1000 lux, de sorte que la variation de l'indicateur de confort soit limitée à une perte de 2 points.

L'éclairement reçu par le porteur après la variation doit alors être au maximum de 6000 lux, ce qui correspond à une transmission du filtre inférieure ou égale à 40%.

Lors des variations d'intensité lumineuse positives, le confort visuel et les performances visuelles sont affectées. Dans le cadre de variations négatives de lumière, le confort visuel est optimal, par contre cette baisse de luminosité affecte davantage les performances visuelles du sujet. Il doit s'adapter à une diminution de l'éclairement rétinien. Le sujet peut perdre entre autre en acuité visuelle, en sensibilité aux contrastes. Un temps de récupération de la vision est présent jusqu'à ce que les processus rétiniens soient régénérés.

Un exemple particulier du procédé selon l'invention s'attache à caractériser cette baisse de vision en lien avec la détermination des zones dynamiques du cône de prescription.

Ainsi, le procédé comporte en outre une étape d'évaluation de l'impact de ladite variation de flux lumineux sur les performances visuelles du porteur.

Après avoir augmenté l'éclairement depuis une valeur initiale EiC jusqu'à la valeur seuil de confort d'éclairement ESC pour différente variations du flux lumineux, c'est-à-dire différentes variations d'intensité globale et différentes durées de variations, on applique une diminution brusque de l'éclairement pour atteindre une valeur d'éclairement minimale Emin de 13 lux.

On réalise ensuite un test d'acuité visuelle, définie comme la capacité à discriminer un opto-type sous le plus petit angle, telle que décrite dans l'ouvrage BORISH'S CLINICAL REFRACTION, (Buttermorth-Heinemann; 2nd Edition, October 27, 2006),

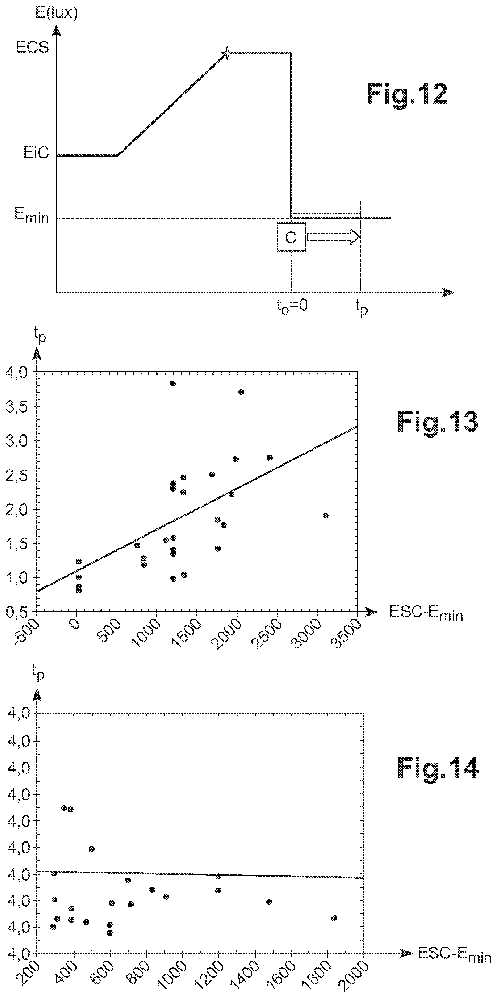

La variation d'éclairement du flux lumineux pendant ce test est représenté sur la figure 12.

Selon la valeur seuil de confort d'éclairement, l'amplitude de la baisse est différente. Une lettre d'acuité 2/10 et de contraste de 10% est affichée au fond de la coupole lorsque la baisse de lumière est appliquée (instant $t_0 = 0$ sur la figure 12). On relève le temps que le met le porteur à retrouver la vision de la lettre (instant $t_p$ sur la figure 12). La lettre est un C de Landolt dont l'ouverture est positionnée aléatoirement. Le porteur doit indiquer la direction de l'ouverture. On note le temps de réponse en seconde pour une identification juste de l'ouverture de la lettre. Il s'agit ici d'un autre type de test de sensibilité adaptométrique.

Deux exemples de résultats sont représentés sur les figures 13 et 14. Ils montrent la corrélation entre le temps de récupération tp de la vision en secondes et l'amplitude de la baisse d'éclairement subie égale à l'écart entre la valeur seuil d'éclairement de confort ESC et la valeur minimale d'éclairement Emin atteinte pour deux sujets différents.

On établit ici une relation affine entre temps de récupération et écart d'éclairement.

Pour la figure 13, cette relation affine s'écrit : tp =1,2814+ 0,0005*(ESC-Emin).

Pour la figure 14, cette relation affine s'écrit : tp = 8,313 - 0,0003*(ESC-Emin).

Pour le porteur dont les données sont représentées sur la figure 13, plus l'amplitude de la baisse d'éclairement est importante, plus le sujet a besoin de temps pour retrouver une vision optimale.

Pour le porteur dont les données sont représentées sur la figure 14, le porteur présente un temps de récupération pratiquement constant, quel que soit la baisse d'éclairement lumineux.

Lorsque le porteur porte un équipement optique, la baisse d'éclairement subie est liée à la fois à la variation du flux lumineux incident et à la présence d'un filtre disposé devant ses yeux. A l'étape b), la transmission du filtre peut alors être ajustée afin de réduire l'amplitude de la baisse d'éclairement subie. En pratique, il s'agit d'augmenter cette transmission.

Dans le cas du porteur de la figure 13, il est préconisé un filtre adaptatif à l'environnement lumineux (photochromique ou électrochromique) passant de l'état foncé à l'état clair le plus rapidement possible. Un temps de passage de l'état foncé à l'état clair de 1 à 2 secondes est acceptable.

Dans le cas d'un filtre photochromique, on proposera un filtre présentant un passage à l'état clair rapide.

Dans le cas d'un filtre électrochromique, on proposera une transmission limitant la baisse d'éclairement perçue par le porteur à 1500 lux.

On peut également proposer un filtre dont la teinte est dégradée si la transmission déterminée pour le filtre n'est pas suffisante pour optimiser la vision.

Un tel filtre présente ainsi une variation de préférence continue de sa transmission entre une partie supérieure et une partie inférieure, situées par rapport à leur position devant les yeux du porteur.

Un filtre ayant une teinte foncée dans sa partie supérieure et une teinte claire dans sa partie inférieure permettra, par exemple, de percevoir les trottoirs, les dénivelés, par exemple, plus facilement et éviter le risque de chute pour les seniors..

En variante, lors de l'étape d'évaluation de l'impact de la variation de flux lumineux sur les performances visuelles du porteur, on réalise au moins une mesure de l'une des grandeurs suivantes sur le porteur :

- la sensibilité aux contrastes : capacités du système visuel à détecter des différences de luminance sur des éléments de dimensions variées, statiques (contraste spatial de luminance), ou dynamiques (contraste temporel de luminance), voir par exemple Sidorova et al., (« Functional acuity contrast sensitivity assessment in young and middle age healthy persons at the day time with and without glare », Acta Medica Lituanica, Vol. 21, No. 1, 2014) ,
- le champ de vision qui correspond à l'étendue de l'espace que perçoit l'œil du porteur quand il est fixe et regarde face à lui (*BORISH'S CLINICAL REFRACTION, op. cit.*),
- la perception des couleurs, c'est-à-dire la perception visuelle de la répartition spectrale de la lumière visible. Cette sensation prend son origine dans la stimulation de cellules nerveuses spécialisées nommées cônes et situées sur la rétine (*op. cit*.),
- la perception des distances et des profondeurs. La perception de la profondeur est la capacité visuelle à percevoir le monde en trois dimensions et à discriminer la position d'un objet par rapport à un autre (*op. cit*.),
- le mouvement de paupière qui se caractérise par une fermeture complète ou partielle des paupières, ainsi que des tremblements de paupières suite à une activité musculaire supérieure à celle en position de repos. L'activité musculaire peut être évaluée par son activité électrique (électromyogramme), voir par exemple Murray et al. (« The ocular stress monitor: a new device for measuring discomfort glare », Lighting Research and Technology, September 2002, 34:240),
- le diamètre pupillaire : taille de l'orifice circulaire situé au centre de l'iris et permettant, par sa contraction ou sa dilatation, de doser la quantité de lumière qui pénètre dans l'œil (cf. Alexandridis E., « The Pupil ». Springer; 1985), et d'autre caractéristiques pupillaires comme la forme de la pupille,
- l'inconfort visuel sur une échelle d'inconfort : gêne ou malaise éprouvé par rapport à une sensation suite à des stimuli lumineux intenses (Mainster et al., « Glare's causes, consequences, and clinical challenges after a century of ophthalmic study ». Am. J. Ophthalmol., 153 (4), pp. 587-593. 2012), et
- le temps de récupération après éblouissement : temps nécessaire à la récupération de tout ou partie des fonctions qui ont été dégradées pendant un éblouissement (Shieber, « Age and Glare Recovery Time for Low-Contrast Stimuli Effect of glare on reaction time for peripheral vision at mesopic adaptation »; Proceedings of the Human Factors and Ergonomics Society Annual Meeting October 1994, 38:496-499).

La connaissance des habitudes d'exposition lumineuse du porteur (passées et futures) associée aux mesures d'adaptation à l'obscurité et de seuil de photosensibilité permettent de connaître le type de variation de flux lumineux auxquelles le porteur est soumis, de connaître la valeur seuil d'intensité, de luminance ou d'éclairement de confort du porteur, de définir le meilleur filtre, à savoir la meilleure combinaison entre filtrage spectral, niveau d'obscurcissement photochromique et temps de retour à l'état clair, pour que le porteur soit à la fois protégé de l'éblouissement et qu'il conserve une bonne performance visuelle.

Selon une deuxième famille de procédé, la détermination de la grandeur relative à la sensibilité dynamique de l'œil du porteur est réalisée à partir d'au moins une information mesurée ou collectée sur la base d'un questionnaire relative aux habitudes d'exposition à la lumière du porteur. Cette famille comprend l'exemple 5.

## EXEMPLE 5

Dans cet exemple, l'étape a) comprend les sous-étapes suivantes :

a3) une étape de soumission du porteur à un questionnaire permettant d'apprécier la sensibilité du porteur à ladite variation de flux lumineux,
a4) une étape de collecte des réponses du porteur audit questionnaire.

Alors, à l'étape a), ladite grandeur représentative d'une sensibilité dynamique de l'œil ou des deux yeux du porteur à une variation d'un flux lumineux est déterminée en tenant compte des réponses au questionnaire collectées à l'étape a3).

En pratique, on propose au porteur un questionnaire permettant de déterminer la sensibilité dynamique de ses yeux aux variations de flux lumineux.

On propose un ensemble de questions pour lequel le porteur fournit un indicateur sur son niveau de confort visuel ou de qualité visuelle, pour différentes variations de flux lumineux, etc... et en fonction de ses activités, par exemple conduite, lecture, activité sportive, activité en intérieur ou en extérieur.

Le questionnaire doit ici de préférence prendre en compte trois phases temporelles différentes : par rapport à un temps donné t de l'accueil du porteur, des informations sont récupérées sur :

- les habitudes d'exposition lumineuse du porteur à un environnement donné avant le temps t donné,
- l'analyse de la sensibilité et de l'adaptation du sujet à la dynamique du flux lumineux au temps t,
- les habitudes de vie et l'environnement lumineux dans lequel évoluera le porteur.

L'état de sensibilité dynamique de la rétine à un temps t aura un impact sur la sensation d'éblouissement consécutive à un changement d'intensité lumineuse.

Par exemple, si le sujet est sujet à des expositions chroniques d'intensité faibles, sa sensibilité à la lumière sera plus importante. Le besoin de protection sera ainsi différent et une prescription de filtre avec une transmission plus faible sera conseillée.

Les études de la demanderesse ont en outre montré que le confort visuel suite à une variation du flux lumineux reçu par le porteur est dépendant des paramètres suivants :

- une amplitude de la variation d'intention de la lumière subie,
- une intensité initiale du flux lumineux avant la variation du flux lumineux.

Plus précisément, plus l'intensité du flux lumineux avant la variation est élevée et plus un éclairement rétinien du porteur avant cette variation est élevé, moins le confort visuel du porteur est diminué après la variation du flux lumineux.

En pratique, le questionnaire permet de collecter des informations concernant différentes périodes : passé, présent, futur, sur une échelle variable : heures, semaines, mois.

Plus précisément, il permet notamment de collecter des informations sur les habitudes d'exposition à la lumière du porteur concerné :

- caractéristiques des sources lumineuses auxquelles il est exposé : lumière artificielle (par exemple LED ou ampoule à incandescence) ou lumière naturelle, lumière diffuse ou ponctuelle ;
- durée d'exposition : instantanée, courte (quelques secondes ou minutes), longue (quelques heures), continue ou intermittente ;
- lieu géographique de vie du porteur ;
- climat du lieu géographique de vie du porteur et notamment durée d'ensoleillement moyenne ;
- activités pratiquées/métier : cette information a des implications sur la durée d'exposition lumineuse et les caractéristiques des sources lumineuses selon que les activités se déroulent à l'intérieur et/ou à l'extérieur: intensités, spectres, variations lumineuses.

Par exemple, une personne qui travaille à la mine toute la journée, dans un environnement fermé, à lumière artificielle, d'intensité faible habituera sa rétine à ce flux faible. Sa sensibilité à la lumière et aux variations de flux lumineux sera plus importante lorsqu'il sera confronté à un environnement lumineux extérieur donné. A l'opposé, un ouvrier du bâtiment, travaillant toute la journée à l'extérieur, pour un même niveau lumineux que le précédent, sera moins gêné. Si les yeux du porteur sont adaptés à l'obscurité, leur sensibilité à une variation de flux lumineux sera plus importante.

Le questionnaire permet également de collecter des informations objectives et subjectives sur le porteur concerné :

- âge,
- sensibilité générale à la lumière,
- sensibilité à la lumière en fonction des conditions lumineuses (intérieur, extérieur, nuit, ...
- sensibilité aux variations de flux lumineux : variation temporelle ou spatiale de l'intensité du flux lumineux, variation temporelle ou spatiale du spectre du flux lumineux, pour des variations positives indiquant une augmentation du flux lumineux, par exemple un passage d'une zone d'obscurité vers une zone illuminée et pour des variations négatives indiquant une diminution du flux lumineux, par exemple un passage d'une zone illuminée vers une zone d'obscurité,
- présence de pathologie visuelle, neurologique (affectant la sensibilité à la lumière du sujet), opération de la cataracte et type de cristallin artificiel implanté, par exemple cristallin artificiel jaune, filtrant le bleu ou blanc,
- performances visuelles et confort visuel exprimés par l'échelle subjective d'évaluation décrite ci-dessus, en fonction de conditions lumineuses données,

- utilisation régulière de lunette de soleil : ponctuel, continu, en fonction des conditions lumineuses, et appréciation sur les lunettes portées.

Ce questionnaire est rempli soit chez l'opticien, par celui-ci ou par le porteur, soit chez le porteur par celui-ci, de manière régulière.

Par ailleurs, le remplissage de ce questionnaire peut être fait en temps réel, par le porteur, dans des conditions lumineuses données : le porteur répond par exemple à une ou plusieurs questions permettant de caractériser à l'instant présent son confort visuel et/ou ses performances visuelles.

Il peut s'agir par exemple d'une question s'affichant sur son smartphone ou sa tablette.

En parallèle, l'étape a) peut comprendre une étape de mesure du flux lumineux auquel est habituellement soumis le porteur. Elle est réalisée à l'aide d'un capteur de flux lumineux indépendant ou intégré à une paire de lunettes ou un objet connecté du porteur, par exemple un smartphone, une tablette ou une montre connectée qui recueillent les caractéristiques du flux lumineux ambiant à cet instant présent. Ce capteur (type spectrophotomètre) permet de recueillir les caractéristiques du flux lumineux auquel est soumis le porteur pendant qu'il remplit le questionnaire (notamment intensité, spectre, variation dans le temps).

En variante de ce questionnaire, on peut envisager que les informations relatives aux habitudes d'exposition du porteur, notamment les caractéristiques des sources lumineuses auquel il est exposé et la durée d'exposition, sont directement mesurées par ces capteurs embarqués sur le porteur et qui effectuent des mesures en continue ou à intervalles de temps prédéterminés.

À titre d'exemple, la question posée au porteur peut consister en nue évaluation subjective de son confort visuel et/ou de ses performances visuelles. Le porteur peut fournir un indicateur de confort subjectif compris entre 1 et 5 sur une échelle d'évaluation standardisée.

Sur cette échelle, les différents indicateurs sont les suivants :

- niveau « 1 » : niveau de confort visuel insupportable ou niveau de qualité visuelle très mauvais ;
- niveau « 2 » : niveau de confort visuel dérangeant ou niveau de qualité visuelle médiocre ;
- niveau « 3 » : niveau de confort visuel juste supportable ou niveau de qualité visuelle juste acceptable ;
- niveau « 4 » : niveau de confort visuel ou de qualité visuelle satisfaisant ;
- niveau « 5 » : niveau de confort visuel ou de qualité visuelle excellent.

L'inconfort visuel est défini comme une sensation subjective d'inconfort visuel liée à la quantité, à la distribution et à la qualité de la lumière reçue. L'échelle d'inconfort visuel correspond à une gradation progressive de l'expression de son inconfort visuel selon différents critères (Gellatly and Weintraub, « User reconfigurations of the de boer rating for discomfort glare », 1990).

On peut ainsi déterminer en fonction des réponses un profil de sensibilité dynamique du porteur aux variations de flux lumineux.

Il est alors possible de déterminer la grandeur relative à la sensibilité dynamique du porteur de différentes manières.

Selon une première méthode, on peut envisager avoir une base de données de porteurs de filtres pour lesquels la sensibilité dynamique des yeux ont été mesurés, par exemple selon un protocole tel que décrit dans l'un des exemples développés ci-dessous et pour lesquels les profils de sensibilité dynamique ont été déterminés avec un questionnaire identique.

La grandeur relative à la sensibilité dynamique des yeux du porteur est alors déterminée à partir d'une grandeur de référence relative à la sensibilité dynamique des yeux des porteurs de la base de données ayant le même profil de sensibilité dynamique, par exemple par identification avec cette grandeur de référence.

A l'étape b), le filtre proposé est déterminé par exemple suivant les exemples développés ci-dessous.

Selon une seconde méthode, on peut considérer que l'indicateur de confort subjectif exprimé par le porteur pour les différentes conditions de variations lumineuses et les différentes activités comme étant une mesure directe de sa sensibilité dynamique. La grandeur relative à la sensibilité dynamique du porteur est alors directement égale à cette indicateur, ou déterminée en fonction de celui-ci.

Ainsi, par exemple, si le porteur exprime un inconfort pour une question portant sur une variation lumineuse données, alors le niveau de transmission du filtre peut être déterminé directement par l'indicateur de confort pour cette variation.

Alors, à l'étape b), par exemple, pour un indicateur de confort de niveau de « 1 » pour une variation de l'intensité du flux lumineux courante sur l'échelle décrite précédemment, on détermine un filtre ayant une transmission de 10%. À l'inverse, pour un indicateur de confort de niveau « 5 » (aucune gêne, confort excellent), on détermine un filtre ayant une transmission de 90%.

Pour un porteur qui vit à l'intérieur la semaine et sort beaucoup le week-end à l'extérieur, présentant une sensibilité exprimée importante à la lumière et aux variations de flux lumineux, un filtre dont la transmission le place dans la classe 3, et/ou polarisé est à recommander pour une utilisation le week-end.

Pour un porteur qui vit fréquemment à l'extérieur, sans inconfort exprimé lors des variations de flux lumineux, un filtre passif avec un transmission le plaçant dans l'une des classes 1 ou 2 est déterminé à l'étape b).

Pour un porteur qui vit fréquemment à l'extérieur, avec un inconfort exprimé important lors des variations de flux lumineux, une sensibilité à la lumière importante, et éventuellement une diminution des performances visuelles allant jusqu'à la perte de vision après une variation du flux lumineux, un filtre de préférence actif présentant une transmission le plaçant dans la classe 3 est déterminé.

Il s'agit par exemple d'un filtre présentant des propriétés photochromiques ou électrochromiques autorisant le passage d'un état clair à un état foncé du filtre correspondant à deux niveaux différents de transmission de la lumière à au moins une longueur d'onde.

Le filtre déterminé ici présente de plus de un éclaircissement rapide, c'est-à-dire un temps de passage de l'état foncé vers l'état clair court.

Ce filtre sera éventuellement polarisé pour améliorer le confort du porteur à haute luminosité.

On peut également déterminer la nécessité de port d'un filtre coloré lors d'une activité en intérieur: dans une salle de classe, sur un poste de travail, utilisation d'écrans.

Dans tous les cas, les réponses au questionnaire peuvent être pondérées en fonction du porteur ou en fonction de la fréquence à laquelle il rencontre la situation correspondant à la question.

Par exemple, si un porteur passe plus de temps en extérieur qu'en intérieur, on propose un poids plus élevé aux questions portant sur les conditions lumineuses extérieures.

À cet effet, on peut demander au porteur d'associer, pour chaque question, un coefficient donnant la fréquence à laquelle la situation est rencontrée, par exemple un coefficient 1 pour une situation rare, un coefficient 2 pour une situation occasionnelle, un coefficient 3 pour une situation fréquente, et un coefficient 4 pour une situation très fréquente.

De manière générale, quelle que soit la méthode utilisée (exemples 1 à 5), la détermination du filtre suivant l'une des méthodes décrites peut impliquer d'utiliser un filtre dont la transmission varie spatialement sur la lentille ophtalmique.

En effet, les sources lumineuses et les variations de flux lumineux pouvant se situer dans des directions privilégiées dans l'environnement du porteur, on peut envisager d'avoir un filtre présentant un taux d'absorption et/ou une réponse spectrale différent entre la partie supérieure et la partie inférieure de la lentille ophtalmique.

D'un côté, la partie supérieure est principalement utilisée pour une activité en extérieur où le flux lumineux peut être très élevé et où le spectre de ce flux est celui de la lumière naturelle.

De l'autre côté, la partie inférieure est principalement utilisée pour une activité intérieure où le flux lumineux est limité et où le spectre de ce flux est souvent celui d'une lumière artificielle.

On notera enfin que les différentes méthodes des exemples 1 à 5 peuvent être combinées entre elles afin d'affiner la détermination du filtre optique.

Il est notamment possible de combiner la méthode des pigments maculaires (exemple 2) avec un questionnaire (exemple 5) de manière à obtenir un profil de sensibilité à la lumière de l'œil du porteur qui soit plus précis et plus complet.

En outre la détermination du filtre à l'étape b) peut prendre en compte les caractéristiques du flux lumineux habituel ou courant (mesuré en temps réel) entourant le porteur.

En particulier, la détermination du filtre à l'étape b) peut prendre en compte les grandeurs et valeurs de paramètres relatifs au porteur déterminés dans le cadre de l'étape a), relatifs par exemple à la pupille du porteur, à l'éclairement rétinien, à la sensibilité statique et dynamique du porteur pour différentes conditions et variations de flux lumineux données.

Elle prend également en compte, de préférence des valeurs de paramètres environnementaux liés aux caractéristiques du flux lumineux dans l'environnement du porteur.

Cela est notamment possible grâce à l'intégration de capteurs notamment spectrophotométriques, sur une paire de lunettes comportant des lentilles ophtalmiques à filtre actif. Ces capteurs mesurent la quantité cumulée du flux lumineux (éclairement, luminance,..) reçue en fonction de la longueur d'onde et enregistrent l'évolution de l'éclairement au cours du temps (de la journée, semaines). Cela permet de contrôler et de personnaliser ledit filtre actif. Les quantités cumulées par longueur d'onde peuvent être comparées pour asservir le changement de transmission du filtre en fonction de l'éclairement extérieur mais également des éclairements cumulés sur plusieurs jours.

Enfin, l'identification des déplacements du porteur par exemple grâce à d'autres capteurs tels qu'un accéléromètre ou un GPS, peut permettre de prévoir les variations de flux lumineux incident sur la rétine dans le temps et d'anticiper l'activation du filtre actif. Afin d'anticiper les variations temporelles de flux lumineux du porteur, une analyse de scène photométrique sera défini par les capteurs intégrés afin d'anticiper l'activation des caractéristiques du filtre selon le profil de sensibilité du porteur.

Ces capteurs (forme, taille) sont de préférence intégrés à la monture de lunettes de manière à analyser le comportement des pupilles d'un porteur dans un champ de vision > 30°, et d'explorer l'environnement du porteur sur plus de 180° dans le champ horizontal et sur plus de 90° dans le champ vertical, sur une profondeur d'analyse d'au moins 5 mètres.

Dans le cas de filtre actifs décrits ici, la monture de lunettes peut également intégrer l'unité de traitement informatique programmée pour effectuer un procédé équivalent à celui selon l'invention, de manière à déterminer les caractéristiques du filtre électrochrome.

De manière plus générale, les grandeurs et/ou paramètres mesurées/déterminés à l'étape a) peuvent comprendre un indicateur de confort donné en temps réel par le porteur pour adapter le filtre, ou être déterminés de manière automatique selon l'analyse objective du comportement du porteur (analyse de la pupille, des mouvements de paupière, indicateur d'inconfort, mouvement de tête ...).

Ces informations peuvent être en outre utilisées pour modifier les règles de choix du filtre utilisées à l'étape b) par apprentissage. L'enregistrement des habitudes et expériences d'expositions seront effectués pour continuer à affiner l'algorithme en continu et selon le mode de vie des porteurs (boucle continue). Par extension, les verres initialement fournis au porteur pourraient être "standards", par exemple optimisés pour un porteur moyen, leur personnalisation ne s'effectuant que par apprentissage.

## Revendications

1. Procédé de fabrication d'un filtre pour une lentille ophtalmique ou d'une lentille ophtalmique comportant un tel filtre, ladite lentille étant destinée à être placée devant l'œil d'un porteur, ledit filtre étant apte à améliorer ou à maintenir le confort visuel et/ou les performances visuelles dudit porteur, et présentant des propriétés photochromiques ou électrochromiques autorisant le passage d'un état clair à un état foncé du filtre correspondant à deux niveaux différents de transmission de la lumière à au moins une longueur d'onde,
   **caractérisé en ce qu'**il comporte :

   a) une étape de détermination d'une grandeur représentative d'une sensibilité dynamique de l'œil ou des deux yeux du porteur à une variation d'un flux lumineux, et
   b) une étape, mise en œuvre au moyen d'une unité de traitement informatique, de détermination d'au moins une caractéristique optique dudit filtre en fonction de la grandeur représentative déterminée, ladite caractéristique optique étant un temps de passage de l'un à l'autre desdits état clair et état foncé, et de commande de la fabrication dudit filtre et/ou de la lentille ophtalmique munie dudit filtre.

2. Procédé selon la revendication 1, selon lequel ladite grandeur représentative de la sensibilité dynamique de l'œil du porteur à la variation du flux lumineux est représentative de l'évolution du confort visuel et/ou des performances visuelles du porteur en fonction de la variation du flux lumineux.

3. Procédé selon l'une des revendications précédentes, selon lequel à l'étape b), on détermine le niveau de transmission d'au moins un desdits états clair et foncé en fonction de la sensibilité dynamique du porteur aux variations de flux lumineux.

4. Procédé selon l'une des revendications précédentes, selon lequel à l'étape b), on détermine ledit temps de passage nécessaire pour passer de l'un à l'autre des états clair et foncé comme étant d'autant plus court que la grandeur représentative de la sensibilité dynamique de l'œil du porteur déterminée à l'étape a) indique une capacité d'adaptation faible vis-à-vis des variations négatives de l'intensité du flux lumineux.

5. Procédé selon l'une des revendications précédentes, selon lequel ladite grandeur représentative de la sensibilité dynamique de l'œil du porteur à la variation du flux lumineux correspond à un temps d'adaptation de l'œil à la variation de ce flux lumineux.

6. Procédé selon l'une des revendications précédentes, selon à l'étape a), ladite grandeur représentative de la sensibilité dynamique de l'œil du porteur à la variation du flux lumineux correspond à une vitesse seuil de confort et/ou une variation de seuil de confort du porteur pour la variation de flux lumineux et, à l'étape b), ledit temps de passage entre ces deux états est déterminé en fonction de cette vitesse seuil de confort et/ou une variation de seuil de confort.

7. Procédé selon l'une des revendications précédentes, selon lequel à l'étape a), ladite grandeur représentative de la sensibilité dynamique de l'œil du porteur à la variation du flux lumineux correspond à une valeur seuil de confort pour l''intensité lumineuse perçue par le porteur lors de la variation de flux lumineux et, à l'étape b), le niveau de transmission de l'état clair et/ou foncé du filtre est déterminé en fonction de cette valeur seuil de confort.

8. Procédé selon l'une des revendications précédentes, selon lequel ladite grandeur représentative de la sensibilité dynamique de l'œil du porteur à la variation du flux lumineux est déterminée en tenant compte d'au moins un des paramètres suivants :

- un paramètre relatif aux habitudes d'exposition à la lumière du porteur passées, présentes et/ou futures,
- un paramètre relatif à la sensibilité statique au flux lumineux du porteur,
- un paramètre relatif à une amplitude de la variation temporelle et/ou spatiale d'intensité et/ou de spectre du flux lumineux,
- un paramètre subjectif relatif aux performances visuelles du porteur pour des conditions lumineuses et/ou de variation lumineuse données,
- un paramètre subjectif relatif au confort visuel pour des conditions lumineuses et/ou de variations lumineuses données,
- un paramètre lié à l'âge du porteur,
- un paramètre relatif à l'utilisation de lunettes de soleil,
- un paramètre lié à un coefficient de diffusion intraoculaire de l'œil du porteur,
- un paramètre lié à une densité et/ou à une répartition du pigment maculaire de l'œil du porteur,
- un paramètre lié à une capacité d'adaptation de la rétine à la lumière ou à l'obscurité,
- un paramètre lié à une dynamique de réponse pupillaire à la variation lumineuse et/ou à une autre caractéristique pupillaire,
- un paramètre relatif à une pathologie visuelle ou une anomalie oculaire éventuelle du porteur,
- un paramètre lié à un seuil de variation du confort visuel et/ou des performances visuelles exprimé ou mesuré.

9. Procédé selon l'une des revendications précédentes, selon lequel l'étape a) comprend une étape de mesure du flux lumineux dynamique auquel est habituellement soumis le porteur.

10. Procédé selon l'une des revendications précédentes, selon lequel, à l'étape a), la variation du flux lumineux comprend au moins :

- une variation temporelle et/ou spatiale d'une intensité dudit flux lumineux et/ou
- une variation temporelle et/ou spatiale d'un spectre dudit flux lumineux et/ou
- une variation dans l'espace d'une distribution spatiale dudit flux lumineux et/ou
- une variation dans l'espace d'une distribution angulaire dudit flux lumineux.

11. Procédé selon l'une des revendications précédentes, selon lequel on réalise en outre une étape de détermination d'une grandeur représentative de l'environnement dans lequel le filtre est utilisé par le porteur et ladite caractéristique optique dudit filtre est déterminée tenant compte de cette grandeur représentative de l'environnement.

12. Procédé selon l'une des revendications précédentes, selon à l'étape a), ladite grandeur représentative de la sensibilité dynamique de l'œil du porteur à la variation du flux lumineux correspond à une vitesse seuil de confort et/ou une variation de seuil de confort du porteur pour la variation de flux lumineux et, à l'étape b), un écart de transmission entre les états clair et foncé et/ou une vitesse de passage de l'un à l'autre des états clair et foncé du filtre est en outre déterminée en fonction de cette vitesse seuil de confort et/ou une variation de seuil de confort.

13. Procédé selon l'une des revendications précédentes, selon lequel à l'étape b), on détermine le temps de passage de l'un à l'autre des états clair et foncé d'autant plus court que la sensibilité dynamique de l'œil ou des deux yeux du porteur déterminé à l'étape a) indique une capacité d'adaptation faible.

**Patentansprüche**

1. Verfahren zur Herstellung eines Filters für eine ophthalmische Linse oder einer einen solchen Filter umfassenden ophthalmischen Linse, wobei die Linse dazu bestimmt ist, vor dem Auge eines Trägers angeordnet zu werden, wobei der Filter in der Lage ist, den Sehkomfort und/oder die Sehleistungen des Trägers zu verbessern oder aufrechtzuerhalten, und photochrome oder elektrochrome Eigenschaften aufweist, die den Übergang von einem hellen Zustand zu einem abgedunkelten Zustand des Filters zulassen, die zwei verschiedenen Transmissionsgraden des Lichts bei mindestens einer Wellenlänge entsprechen; **dadurch gekennzeichnet, dass** es umfasst:

a) einen Schritt des Ermittelns einer für eine dynamische Empfindlichkeit des Auges oder der beiden Augen des Trägers gegenüber einer Änderung eines Lichtstroms repräsentativen Größe, und

b) einen mittels einer Datenverarbeitungseinrichtung durchgeführten Schritt des Ermittelns mindestens eines optischen Merkmals des Filters in Abhängigkeit von der ermittelten repräsentativen Größe, wobei das optische Merkmal eine Übergangszeit von dem einen zu dem anderen des hellen Zustands und des abgedunkelten Zustands ist, und des Steuerns der Herstellung des Filters und/oder der mit dem Filter versehenen ophthalmischen Linse.

2. Verfahren nach Anspruch 1, bei dem die für die dynamische Empfindlichkeit des Auges des Trägers gegenüber einer Änderung des Lichtstroms repräsentative Größe für die Entwicklung des Sehkomforts und/oder der Sehleistungen des Trägers in Abhängigkeit von der Änderung des Lichtstroms repräsentativ ist.

3. Verfahren nach einem der vorhergehenden Ansprüche, bei dem im Schritt b) der Transmissionsgrad mindestens eines der hellen und abgedunkelten Zustände in Abhängigkeit von der dynamischen Empfindlichkeit des Trägers gegenüber Änderungen des Lichtstroms ermittelt wird.

4. Verfahren nach einem der vorhergehenden Ansprüche, bei dem im Schritt b) die erforderliche Übergangszeit zum Übergehen von dem einen zu dem anderen der hellen und abgedunkelten Zustände so ermittelt wird, dass sie umso kürzer ist, je stärker die im Schritt a) ermittelte dynamische Empfindlichkeit des Auges des Trägers auf eine schwache Anpassungsfähigkeit gegenüber negativen Änderungen der Intensität des Lichtstroms hindeutet.

5. Verfahren nach einem der vorhergehenden Ansprüche, bei dem die für die dynamische Empfindlichkeit des Auges des Trägers gegenüber der Änderung des Lichtstroms repräsentative Größe einer Anpassungszeit des Auges an die Änderung dieses Lichtstroms entspricht.

6. Verfahren nach einem der vorhergehenden Ansprüche, bei dem im Schritt a) die für die dynamische Empfindlichkeit des Auges des Trägers gegenüber der Änderung des Lichtstroms repräsentative Größe einer Komfortschwellengeschwindigkeit und/oder einer Komfortschwellenänderung des Trägers bei der Änderung des Lichtstroms entspricht und im Schritt b) die Übergangszeit zwischen diesen beiden Zuständen in Abhängigkeit von dieser Komfortschwellengeschwindigkeit und/oder einer Komfortschwellenänderung ermittelt wird.

7. Verfahren nach einem der vorhergehenden Ansprüche, bei dem im Schritt a) die für die dynamische Empfindlichkeit des Auges des Trägers gegenüber der Änderung des Lichtstroms repräsentative Größe einem Komfortschwellenwert bei der Lichtintensität entspricht, der von dem Träger bei der Änderung des Lichtstroms wahrgenommen wird, und im Schritt b) der Transmissionsgrad des hellen und/oder abgedunkelten Zustands des Filters in Abhängigkeit von diesem Komfortschwellenwert ermittelt wird.

8. Verfahren nach einem der vorhergehenden Ansprüche, bei dem die für die dynamische Empfindlichkeit des Auges des Trägers gegenüber der Änderung des Lichtstroms repräsentative Größe unter Berücksichtigung mindestens eines der folgenden Parameter ermittelt wird:

- ein Parameter bezüglich der früheren, gegenwärtigen und/oder zukünftigen Lichtexpositionsgewohnheiten des Trägers,
- ein Parameter bezüglich der statischen Empfindlichkeit des Trägers gegenüber dem Lichtstrom,
- ein Parameter bezüglich einer Amplitude der zeitlichen und/oder räumlichen Intensitäts- und/oder Spektrumsänderung des Lichtstroms,
- ein subjektiver Parameter bezüglich der Sehleistungen des Trägers bei gegebenen Licht- und/oder Lichtänderungsbedingungen,
- ein subjektiver Parameter bezüglich des Sehkomforts bei gegebenen Licht- und/oder Lichtänderungsbedingungen,
- ein mit dem Alter des Trägers zusammenhängender Parameter,
- ein Parameter bezüglich der Verwendung von Sonnenbrillen,
- ein mit einem Koeffizienten der intraokularen Streuung des Auges des Trägers zusammenhängender Parameter,
- ein mit einer Dichte und/oder einer Verteilung des Makulapigments des Auges des Trägers zusammenhängender Parameter,
- ein mit einer Anpassungsfähigkeit der Netzhaut an das Licht oder an die Dunkelheit zusammenhängender Parameter,

- ein mit einer Dynamik der Pupillenreaktion bei der Lichtänderung und/oder mit einem anderen Pupillenmerkmal zusammenhängender Parameter,
- ein Parameter bezüglich einer Augenpathologie oder einer etwaigen okulären Anomalie des Trägers,
- ein mit einem geäußerten oder gemessenen Änderungsschwelle des Sehkomforts und/oder der Sehleistungen zusammenhängender Parameter.

9. Verfahren nach einem der vorhergehenden Ansprüche, bei dem der Schritt a) einen Schritt des Messens des dynamischen Lichtstroms umfasst, dem der Träger gewöhnlich ausgesetzt ist.

10. Verfahren nach einem der vorhergehenden Ansprüche, bei dem im Schritt a) die Änderung des Lichtstroms mindestens umfasst:

- eine zeitliche und/oder räumliche Änderung einer Intensität des Lichtstroms und/oder
- eine zeitliche und/oder räumliche Änderung eines Spektrums des Lichtstroms und/oder
- eine Änderung im Raum einer räumlichen Verteilung des Lichtstroms und/oder
- eine Änderung im Raum einer Winkelverteilung des Lichtstroms.

11. Verfahren nach einem der vorhergehenden Ansprüche, bei dem ferner ein Schritt des Ermittelns einer Größe ausgeführt wird, die für die Umgebung repräsentativ ist, in welcher der Filter von dem Träger verwendet wird, und das optische Merkmal des Filters unter Berücksichtigung dieser für die Umgebung repräsentativen Größe ermittelt wird.

12. Verfahren nach einem der vorhergehenden Ansprüche, bei dem im Schritt a) die für die dynamische Empfindlichkeit des Auges des Trägers gegenüber der Änderung des Lichtstroms repräsentative Größe einer Komfortschwellengeschwindigkeit und/oder einer Komfortschwellenänderung des Trägers bei der Änderung des Lichtstroms entspricht und im Schritt b) eine Transmissionsdifferenz zwischen den hellen und abgedunkelten Zuständen und/oder eine Übergangsgeschwindigkeit von dem einen zu dem anderen der hellen und abgedunkelten Zustände des Filters ferner in Abhängigkeit von dieser Komfortschwellengeschwindigkeit und/oder einer Komfortschwellenänderung ermittelt wird.

13. Verfahren nach einem der vorhergehenden Ansprüche, bei dem im Schritt b) die Übergangszeit von dem einen zu dem anderen der hellen und abgedunkelten Zustände als umso kürzer ermittelt wird, je stärker die im Schritt a) ermittelte dynamische Empfindlichkeit des Auges oder der beiden Augen des Trägers auf eine schwache Anpassungsfähigkeit hindeutet.

**Claims**

1. Method for manufacturing a filter for an ophthalmic lens or an ophthalmic lens comprising such a filter, said lens being intended to be placed in front of the eye of a wearer, said filter being able to improve or maintain the visual comfort and/or visual performance of said wearer, and having photochromic or electrochromic properties permitting the filter to pass from a clear state to a darkened state corresponding to two different levels of transmission of light at at least one wavelength,
   **characterized in that** it comprises:

   a) a step of determining a quantity representative of a dynamic sensitivity of the eye or of both eyes of the wearer to a variation in a light flux, and
   b) a step, implemented by means of a computational processing unit, of determining at least one optical characteristic of said filter depending on the determined representative quantity, said optical characteristic being a time of passage from one to the next of said clear state and darkened state, and of inducing manufacture of said filter and/or of the ophthalmic lens equipped with said filter.

2. Method according to Claim 1, wherein said quantity representative of the dynamic sensitivity of the eye of the wearer to the variation in the light flux is representative of the evolution of the visual comfort and/or of the visual performance of the wearer as a function of the variation in the light flux.

3. Method according to either of the preceding claims, wherein, in step b), the transmission level of at least one of said clear and darkened states is determined depending on the dynamic sensitivity of the wearer to variations in light flux.

**4.** Method according to any of the preceding claims, wherein, in step b), said time of passage required to pass from one to the next of the clear and darkened states is determined to be all the shorter as the quantity representative of the dynamic sensitivity of the eye of the wearer, i.e. the quantity determined in step a), indicates a low adaptation capacity with respect to negative variations in the intensity of the light flux.

**5.** Method according to any of the preceding claims, wherein said quantity representative of the dynamic sensitivity of the eye of the wearer to the variation in the light flux corresponds to an adaptation time of the eye to the variation in this light flux.

**6.** Method according to any of the preceding claims, wherein, in step a), said quantity representative of the dynamic sensitivity of the eye of the wearer to the variation in the light flux corresponds to a comfort threshold speed and/or a variation in comfort threshold of the wearer for the variation in light flux and, in step b), said time of passage between these two states is determined depending on this comfort threshold speed and/or a variation in comfort threshold.

**7.** Method according to any of the preceding claims, wherein, in step a), said quantity representative of the dynamic sensitivity of the eye of the wearer to the variation in the light flux corresponds to a comfort threshold value for the light intensity perceived by the wearer during the variation in light flux and, in step b), the transmission level of the clear and/or darkened state of the filter is determined depending on this comfort threshold value.

**8.** Method according to any of the preceding claims, wherein said quantity representative of the dynamic sensitivity of the eye of the wearer to the variation in the light flux is determined while taking into account at least one of the following parameters:

   - a parameter relating to the past, present and/or future light exposure habits of the wearer,
   - a parameter relating to the static sensitivity of the wearer to the light flux,
   - a parameter relating to an amplitude of the spatial and/or temporal variation in intensity and/or spectrum of the light flux,
   - a subjective parameter relating to the visual performance of the wearer under given luminous conditions and/or luminous-variation conditions,
   - a subjective parameter relating to visual comfort under given luminous conditions and/or luminous-variation conditions,
   - a parameter related to the age of the wearer,
   - a parameter relating to the use of sunglasses,
   - a parameter related to an intraocular-scattering coefficient of the eye of the wearer,
   - a parameter related to a density and/or a distribution of the macular pigment of the eye of the wearer,
   - a parameter related to a capacity of the retina to adapt to light or darkness,
   - a parameter relating to a dynamic pupillary response to the luminous variation and/or to another pupillary characteristic,
   - a parameter relating to a visual pathology or to any ocular anomaly that the wearer has,
   - a parameter related to an expressed or measured threshold of variation in the visual comfort and/or visual performance.

**9.** Method according to any of the preceding claims, wherein step a) comprises a step of measuring the dynamic light flux to which the wearer is habitually subjected.

**10.** Method according to any of the preceding claims, wherein, in step a), the variation in the light flux comprises at least:

   - a temporal and/or spatial variation in an intensity of said light flux and/or
   - a temporal and/or spatial variation in a spectrum of said light flux and/or
   - a variation in space of a spatial distribution of said light flux and/or
   - a variation in space of an angular distribution of said light flux.

**11.** Method according to any of the preceding claims, wherein a step of determining a quantity representative of the environment in which the filter is used by the wearer is furthermore carried out and said optical characteristic of said filter is determined taking into account this quantity representative of the environment.

**12.** Method according to any of the preceding claims, wherein, in step a), said quantity representative of the dynamic sensitivity of the eye of the wearer to the variation in the light flux corresponds to a comfort threshold speed and/or a variation in comfort threshold of the wearer for the variation in light flux and, in step b), a difference in transmission between the clear and darkened states, and/or a speed of passage from one to the next of the clear and darkened states of the filter is further determined depending on this comfort threshold speed and/or a variation in comfort threshold.

13. Method according to any of the preceding claims, wherein, in step b), the time of passage from one to the next of the clear and darkened states is determined to be all the shorter as the dynamic sensitivity of the eye or of both eyes of the wearer determined in step a) indicates a low adaptation capacity.

## Fig.1

## Fig.2

Fig.3

Fig.4

Fig.5

Fig.6

Fig.7

Fig.8

Fig.9

Fig.10

Fig.11

Fig.12

Fig.13

Fig.14

## RÉFÉRENCES CITÉES DANS LA DESCRIPTION

**Documents brevets cités dans la description**

- WO 2014079574 A

- WO 2001057583 A

**Littérature non-brevet citée dans la description**

- **ROSENBLUM et al.** Spectral filters in low-vision correction. *Ophthalmic Physiol. Opt.*, 2000, vol. 20 (4), 335-341
- **WOLF-SCHNURRBUSCH et al.** Ethnic differences in macular pigment density and distribution. *Invest. Ophthalmol. Vis. Sci.*, 2007, vol. 48 (8), 3783-3787
- **BERNSTEIN PS**. The value of measurement of macular carotenoid pigment optical densities and distributions in age-related macular degeneration and other retinal disorders. *Vision Res.*, 2010
- **BEATTY S. et al.** *Invest. Ophthalmol. Vis. Sci.*, 2001, vol. 42, 439-446
- **STRINGHAM et al.** Macular pigment and visual performance under glare conditions. *Optom. Vis. Sci.*, 2008, vol. 85 (2), 82-88
- **STRINGHAM et al.** Macular Pigment and Visual Performance in Glare: Benefits for Photostress Recovery, Disability Glare, and Visual Discomfort. *Investigative Ophthalmology & Visual Science*, September 2011, vol. 52, 7406-7415
- **STRINGHAM** ; **STRINGHAM JM** ; **HAMMOND BR et al.** Macular pigment and visual performance under glare conditions. *Optom Vis Sci.*, February 2008, vol. 85 (2), 82-8
- **STRINGHAM JM** ; **HAMMOND BR**. The glare hypothesis of macular pigment function. *Optom Vis Sci.*, September 2007, vol. 84 (9), 859-64
- Macular Pigment and Visual Performance in Glare: Benefits for Photostress Recovery, Disability Glare, and Visual Discomfort. *Investigative Ophthalmology & Visual Science*, September 2011, vol. 52, 7406-7415
- **STRINGHAM JM**. Macular Pigment and Visual Performance in Low-Light Conditions. *Invest Ophthalmol Vis Sci.*, April 2015, vol. 56 (4), 2459-68
- **CREUZOT-GARCHER et al.** Comparison of Two Methods to Measure Macular Pigment Optical Density in Healthy Subjects. *Retina 2014 IOVS*, May 2014, vol. 55 (5), 2941-2947

- **JOHN G. EDWARDS** ; **DAVID A. QUILLEN** ; **LAURA WALTER** ; **D. ALFRED OWENS** ; **GREGORY R. JACKSON** ; **GREGORY R. JACKSON** ; **JOHN G. EDWARDS**. Comparison of AdaptRx and Goldmann-Weekers Dark Adaptometers. *J ocul biol dis inform*, 2008, vol. 1, 7-11
- **LAURA E. WALTER** ; **DAVID A. QUILLEN** ; **JOHN G. EDWARDS** ; **ALFRED OWENS** ; **GREGORY R. JACKSON**. Measurement Error of the AdaptRx Dark Adaptometer for Healthy Adults and AMD Patients. *Cette première phase suit une évolution logarithmique*
- **GAMLIN, MC DOUGAL et al.** Human and macaque pupil responses driven by melanopsin-containing retinal ganglion cells. *Vision Research*, 2007, vol. 47 (7), 946-954
- **BUTTERMORTH-HEINEMANN**. BORISH'S CLINICAL REFRACTION,. 27 October 2006
- **SIDOROVA et al.** Functional acuity contrast sensitivity assessment in young and middle age healthy persons at the day time with and without glare. *Acta Medica Lituanica*, 2014, vol. 21 (1)
- **MURRAY et al.** The ocular stress monitor: a new device for measuring discomfort glare. *Lighting Research and Technology*, September 2002, vol. 34, 240
- **ALEXANDRIDIS E**. The Pupil. Springer, 1985
- **MAINSTER et al.** Glare's causes, consequences, and clinical challenges after a century of ophthalmic study. *Am. J. Ophthalmol.*, 2012, vol. 153 (4), 587-593
- **SHIEBER**. Age and Glare Recovery Time for Low-Contrast Stimuli Effect of glare on reaction time for peripheral vision at mesopic adaptation. *Proceedings of the Human Factors and Ergonomics Society Annual Meeting*, October 1994, vol. 38, 496-499
- **GELLATLY** ; **WEINTRAUB**. *User reconfigurations of the de boer rating for discomfort glare*, 1990